# EUROPEAN PATENT APPLICATION

(11) **EP 4 446 407 A1**
(43) Date of publication of application: **16.10.2024**
(21) Application number: 23762919.1
(22) Date of filing: 01.03.2023
(51) Int. Cl.: C12N 1/21, C12N 15/70, A61K 35/74, A61K 35/741, A61K 48/00, A61P 3/00, C12R 1/19

(54) **ENGINEERED MICROORGANISM FOR TREATING HYPERPHENYLALANINEMIA AND USE THEREOF**

(30) Priority: 02.03.2022 CN 202210200137; 08.09.2022 CN 202211097491
(71) Applicant: Commbio Therapeutics Co., Ltd., Shanghai 201203 (CN)
(72) Inventor: XIANG, Bin, Shanghai 201203 (CN); SUN, Wanju, Shanghai 201203 (CN); LIU, Cuicui, Shanghai 201203 (CN); ZHU, Li, Shanghai 201203 (CN)
(74) Representative: Engelhard, Markus
(86) International application number: PCT/CN2023/079034
(87) International publication number: WO 2023/165521

(57) **Abstract**

An engineered microorganism, which comprises one or more of a gene encoding an aromatic amino acid transaminase, a gene encoding phenylalanine dehydrogenase, a gene encoding phenylpyruvate decarboxylase, a gene encoding an aldehyde reductase, a gene encoding glutamate dehydrogenase, and a gene encoding a phenylalanine transport protein, or a functional equivalent thereof. The present invention also relates to a composition comprising the engineered microorganism; a method for using the engineered microorganism or the composition to alleviate and/or treat diseases and/or conditions associated with hyperphenylalaninemia; and use of the engineered microorganism or the composition in the preparation of medicaments or health products for treating diseases and/or conditions associated with hyperphenylalaninemia.

## Description

### TECHNICAL FIELD

The present disclosure relates to the field of genetic engineering, specifically to an engineered microorganism and an engineered microorganism composition, a composition comprising the engineered microorganism or the engineered microorganism composition, and a method of using the engineered microorganism or the engineered microorganism composition to alleviate and/or treat diseases and/or conditions associated with hyperphenylalaninemia, and the use of the engineered microorganism or the engineered microorganism composition in the preparation of a drug or a health product for alleviating or treating diseases and/or conditions associated with hyperphenylalaninemia.

### BACKGROUND ART

Hyperphenylalaninemia (HPA) refers to a type of congenital phenylalanine metabolism disorders resulting from high levels of phenylalanine (Phe) in the blood due to defects in enzymes in the phenylalanine metabolism pathway, such as mutations in phenylalanine hydroxylase (PAH), tetrahydrobiopterin synthase or dihydrobiopterin reductase. Among them, phenylketonuria (PKU) is caused by mutations in phenylalanine hydroxylase (PAH) in the phenylalanine metabolism pathway, which prevents the conversion of phenylalanine to tyrosine, resulting in the accumulation of phenylalanine and α-ketoglutarate in the blood and tissues, which is excreted in large quantities in the urine. The content of phenylalanine in the blood of healthy people is around 100 µM. It is generally believed that when the content of phenylalanine in human blood is above 360 µM - 600 µM, symptoms of phenylketonuria will occur. In China, the incidence of phenylketonuria among newborns is about 1/11,000. If a child with phenylketonuria is not diagnosed and treated in time at birth, it will seriously affect the child's intellectual development and cause symptoms such as epilepsy and movement disorders, and be accompanied by melanin synthesis disorders.

The current treatment for this disease is mainly food control. In patients with phenylketonuria, dietary intervention or treatment can maintain serum phenylalanine levels in the range of 120-360 µM, which may help alleviate disease-related cognitive impairment. However, this treatment often affects the growth of newborns or adolescents, as well as the development of the fetuses in the belly of pregnant women; dietary therapy requires long-term, uninterrupted treatment, which imposes a huge financial burden on patients and their families. In addition to dietary control, approved drugs for treatment of phenylketonuria in recent years include sapropterin hydrochloride and phenylalanine ammonia-lyase. However, sapropterin hydrochloride cannot be used as a standalone treatment and needs to be used in conjunction with dietary control; Phenylalanine ammonia-lyase was recently approved for the treatment of adults with phenylketonuria, relying on systemic injections of phenylalanine ammonia-lyase (PAL). However, this therapy is often accompanied by severe allergic reactions and immune-mediated adverse effects. Scientists are also trying gene therapy by placing a recombinant adenovirus carrying cDNA that expresses the phenylalanine hydroxylase gene (PAH) into mice to restore hepatic PAH activity. However, at present there are mainly problems with inefficient transport efficiency and the high cost of research and development of gene therapy, which makes it an expensive treatment and is of low acceptability for most patients.

Current clinical treatment methods such as dietary control, enzyme cofactors, and enzyme preparations have not achieved good clinical results, and new treatment methods are urgently needed. In attempts to develop new drugs, AAV gene therapy has been suspended from clinical trials by some companies due to showing potential liver carcinogenic toxicity, and approaches to deliver enzyme genes by erythrocytes have been suspended from preclinical studies by some companies due to showing low expression levels. Therefore, there is an urgent need in this field to develop a new and effective drug or health product that can treat or alleviate diseases and/or conditions related to phenylalanine metabolism disorders.

### SUMMARY OF THE INVENTION

In the search for newer and more effective drugs or health products that can treat or alleviate diseases and/or conditions associated with phenylalanine metabolism disorders, the present disclosure attempts to construct engineered microorganisms that can degrade phenylalanine to produce non-toxic intermediate metabolites and can effectively reduce phenylalanine levels in animals (including humans) by using engineered microorganisms as carriers for the delivery of phenylalanine-associated degrading enzymes and utilizing genetic engineering techniques to modify initiating microorganisms (such as intestinal probiotics).

According to an embodiment of the present disclosure, an engineered microorganism into which a exogenous gene is introduced can be provided, characterized in that: the exogenous gene includes: one or more genes encoding an enzyme capable of converting phenylalanine to phenylpyruvate; one or more genes encoding an enzyme capable of converting phenylpyruvate to phenylacetaldehyde; one or more genes encoding an enzyme capable of converting phenylacetaldehyde to phenylethanol; and one or more genes encoding a protein capable of transporting phenylalanine into the engineered microorganism.

According to one embodiment of the present disclosure, an engineered microorganism composition can be provided, which includes more than one engineered microorganism, each of the more than one engineered microorganism independently includes any one or more of the following genes:
a gene encoding an enzyme capable of converting phenylalanine to phenylpyruvate;
a gene encoding an enzyme capable of converting phenylpyruvate to phenylacetaldehyde;
a gene encoding an enzyme capable of converting phenylacetaldehyde to phenylethanol;

Wherein the engineered microorganism composition includes a gene encoding an enzyme capable of converting phenylalanine to phenylpyruvate, a gene encoding an enzyme capable of converting phenylpyruvate to phenylacetaldehyde, and a gene encoding an enzyme capable of converting phenylacetaldehyde to phenylethanol.

According to one embodiment of the present disclosure, a composition can be provided, which includes the engineered microorganism or the engineered microorganism composition according to the present disclosure and a pharmaceutically, nutritionally or physiologically acceptable carrier.

According to one embodiment of the present disclosure, a kit can be provided, which includes the engineered microorganism according to the present disclosure or the engineered microorganism composition according to the present disclosure or the composition according to the present disclosure.

According to one embodiment of the present disclosure, a method for alleviating and/or treating diseases and/or conditions associated with hyperphenylalaninemia can be provided, the method includes administering the engineered microorganism according to the present disclosure or the engineered microorganism composition according to the present disclosure or the composition according to the present disclosure or the kit according to the present disclosure to a patient in need thereof.

According to one embodiment of the present disclosure, the use of the engineered microorganism of the present disclosure or the engineered microorganism composition of the present disclosure or the composition of the present disclosure in the preparation of a drug or a health product for alleviating and/or treating diseases and/or conditions associated with hyperphenylalaninemia can be provided.

### BRIEF DESCRIPTION OF THE DRAWINGS

Fig. 1 depicts the map of plasmid PCBT003.
Fig. 2 depicts the map of plasmid PCBT001.
Fig. 3 depicts the in vitro detection results of phenylalanine degradation by engineered strains EcN, CBT2001, CBT2002 and CBT2003 under aerobic conditions.
Fig. 4 depicts the in vitro detection results of phenylalanine degradation by engineered strains EcN, CBT2001, CBT2002 and CBT2003 under anaerobic conditions.
Fig. 5 depicts the in vitro detection results of phenylalanine degradation by engineered strains CBT209 and CBT210.
Fig. 6 depicts the in vitro detection results of phenylalanine degradation by engineered strain CBT201.
Fig. 7 depicts the in vitro detection results of phenylalanine degradation by engineered strain CBT202.
Fig. 8 depicts the in vitro detection results of phenylalanine degradation by engineered strains CBT205 and CBT206.
Fig. 9 depicts the in vitro detection results of phenylalanine degradation by engineered strains CBT207 and CBT208.
Fig. 10 depicts the in vitro detection results of phenylalanine degradation by engineered strains CBT201, CBT205, CBT207 and CBT211.
Fig. 11 depicts the in vitro detection results of phenylalanine degradation by engineered strains CBT-201, CBT-212, CBT-213, CBT-205 and CBT-207.
Fig. 12 depicts the in vivo detection results of phenylalanine degradation by engineered strains CBT-201, CBT-201-AA, and CBT-205, and negative control group EcN and active reference group SYNB1934.

### DETAILED DESCRIPTION OF EMBODIMENTS

Unless otherwise indicated or contradicts the context, the terms or expressions used herein should be read in conjunction with the entire content of the present disclosure and as understood by those of ordinary skill in the art. All technical and scientific terms used herein have the same meanings as commonly understood by those of ordinary skill in the art, unless otherwise defined.

"Microorganism" as described in the present disclosure refers to microscopic, submicroscopic or ultramicroscopic sized organisms or microorganisms that are typically composed of single cells. Examples of microorganisms include, but are not limited to, bacteria, viruses, parasites, fungi, certain algae, and protozoa. "Engineered microorganism" as described in the present disclosure refers to a microorganism that has been genetically modified to exhibit desired properties or characteristics. Furthermore, in the present disclosure, the term "engineered microorganism" is used interchangeably with "genetically engineered microorganism" or "genetically modified microorganism".

"Phenylalanine" as described in the present disclosure is used to refer to an amino acid having the molecular formula C₆H₅CH₂CH(NH₂)COOH. Phenylalanine is a precursor to tyrosine, dopamine, norepinephrine, and epinephrine. L-phenylalanine is an essential amino acid and is the form of phenylalanine found primarily in dietary proteins; the stereoisomer D-phenylalanine is found to be present in lower amounts in dietary proteins; DL-phenylalanine is a combination of the two forms. Phenylalanine may refer to one or more of L-phenylalanine, D-phenylalanine and DL-phenylalanine.

Unless the context specifically dictates otherwise or is clearly contradicted, references in the present disclosure to "phenylpyruvate" are also intended to encompass phenylpyruvate salts. Unless the context specifically dictates otherwise or is clearly contradicted, "phenylpyruvate decarboxylase" and "phenylpyruvate salt decarboxylase" used in the present disclosure have the same meaning.

"Aromatic amino acid transaminase" as described in the present disclosure refers to a phenylalanine metabolizing enzyme that converts or processes phenylalanine to phenylpyruvate.

"L-amino acid deaminase" as described in the present disclosure refers to a phenylalanine metabolizing enzyme that converts or processes phenylalanine to phenylpyruvate.

"Phenylalanine dehydrogenase" as described in the present disclosure refers to a phenylalanine metabolizing enzyme that converts or processes phenylalanine to phenylpyruvate.

"Phenylalanine metabolizing enzyme" as described in the present disclosure refers to an enzyme capable of degrading or converting phenylalanine. Any phenylalanine metabolizing enzyme known in the art can be encoded by the engineered microorganism. Phenylalanine metabolizing enzymes include, but are not limited to, aromatic amino acid transaminase, phenylalanine dehydrogenase, L-amino acid deaminase, etc.

"Phenylalanine metabolite" as described in the present disclosure refers to metabolites produced due to the degradation of phenylalanine. The metabolite can be produced directly by enzymes that catalyze phenylalanine as a substrate, or the metabolite can be produced indirectly by different enzymes acting downstream of the metabolic pathway of the phenylalanine metabolite. The phenylalanine metabolite can be produced by engineered microorganisms encoding phenylalanine metabolizing enzymes.

"Phenylpyruvate decarboxylase" as described in the present disclosure refers to a phenylpyruvate metabolizing enzyme that converts or processes phenylpyruvate to phenylacetaldehyde.

"α-keto acid decarboxylase" as described in the present disclosure refers to a phenylpyruvate metabolizing enzyme that converts or processes phenylpyruvate to phenylacetaldehyde.

"Aldehyde dehydrogenase" as described in the present disclosure refers to a phenylacetaldehyde metabolizing enzyme that converts or processes phenylacetaldehyde into phenyl ethanol.

"Glutamate dehydrogenase" as described in the present disclosure refers to a glutamate (salt) metabolizing enzyme that converts or processes glutamate or glutamate salt into α-ketoglutarate or α-ketoglutarate salt.

"Phenylalanine transporter" as described in the present disclosure refers to a membrane transporter capable of transporting phenylalanine into cells. In *Escherichia coli,* the phenylalanine transporter gene encodes a high-affinity phenylalanine-specific phenylalanine transporter responsible for phenylalanine transport. The phenylalanine transporter may be encoded by a phenylalanine transporter gene derived from bacteria including, but not limited to, *Acinetobacter calcoaceticus, Salmonella enteritidis,* and *Escherichia coli.* Other phenylalanine transporters include the universal amino acid transporter encoded by the arop gene, which is capable of transporting three aromatic amino acids, including phenylalanine, with high affinity and is thought to be responsible for the majority of phenylalanine inputs along with the phenylalanine transporter. In addition, low-level phenylalanine transport activity has been traced to the activity of the LIV-I/LS system, and the LIV-I/LS system is a branched-chain amino acid transporter consisting of two periplasmic binding proteins, the LIV-binding protein (LIV-I system) and the LS-binding protein (LS system), as well as the membrane component LIVHMGF.

In at least some embodiments, the enzyme capable of converting phenylalanine to phenylpyruvate, the enzyme capable of converting phenylpyruvate to phenylacetaldehyde, the enzyme capable of converting phenylacetaldehyde to phenylethanol, the phenylalanine transporter, and glutamate dehydrogenase, or their encoding genes, of the present disclosure further include the functional equivalents of the above-mentioned enzymes, proteins, or their encoding genes; The term "functional equivalent" refers to any related variant that retains at least partially one or more biological functions of a naturally occurring gene/enzyme, despite differences in amino acid sequence or polynucleotide sequence or chemical structure.

An "expression cassette" as described in the present disclosure refers to a nucleic acid molecule capable of directing the expression of at least one polynucleotide/encoding gene of interest in an appropriate host cell, and in at least some embodiments, the nucleic acid molecule further comprises a promoter and/or other regulatory elements operably linked to the polynucleotide/encoding gene of interest.

"Operably linked" as described in the present disclosure means that a nucleic acid sequence is linked to a regulatory region sequence in a manner that allows expression of the nucleic acid sequence (e.g., acting in cis). The regulatory region is a nucleic acid that can direct the transcription of a target gene, and is for example promoters, and may optionally include enhancers, response elements, protein recognition sites, induction elements, promoter control elements, protein binding sequences, 5' and 3' untranslated regions, transcription start sites, termination sequences, polyadenylation sequences and/or introns.

"Promoter" as described in the present disclosure refers to a polynucleotide sequence that can control the transcription of a coding sequence. Promoter sequences include specific sequences sufficient to enable RNA polymerase to recognize, bind, and initiate transcription. In addition, promoter sequences may include sequences that optionally modulate the recognition, binding and transcription initiation activities of RNA polymerase in the engineered microorganisms provided by the present disclosure. A promoter can affect the transcription of a gene located on the same nucleic acid molecule as the promoter or a gene located on a different nucleic acid molecule from the promoter.

"Inducible promoter" as described in the present disclosure refers to a regulated promoter that can start the increase of the transcription level of coding sequences or genes under its control in one or more cell types through external stimuli, such as chemicals, light, hormones, stress, anaerobic conditions or pathogens. Inducible promoters and variants are well known in the art and include, but are not limited to, PLteto1, galP1, PLlacO1, Pfnrs, PBAD, and Pvan.

"Directly inducible promoter" as described in the present disclosure refers to a regulatory region operably linked to a gene encoding a protein or polypeptide, and the protein or polypeptide is expressed or repressed in the presence of an inducer of the regulatory region.

"Indirectly inducible promoter" as described in the present disclosure refers to a regulatory system comprising two or more regulatory regions, e.g., a first regulatory region operably linked to a gene encoding a first molecule, for example, a transcription regulator, which can regulate a second regulatory region operably linked to a gene encoding an effector molecule. In the presence of an inducer of the first regulatory region, the second regulatory region can be activated or repressed, thereby activating or repressing the expression of the effector molecule. Both directly inducible promoters and indirectly inducible promoters are included in "inducible promoters".

"Constitutive promoter" as described in the present disclosure refers to a promoter capable of promoting continuous transcription of a coding sequence or a gene under its control and/or operably linked thereto. Constitutive promoters and variants of E. coli Nissle1917 (referred to as EcN) are well known in the art, including but not limited to BBa_J23119, BBa_J23101, BBa_J23102, BBa_J23103, BBa_J23109, BBa_J23110, BBa_J23114, BBa_J23117, USP45_promoter, OmpA_promoter, BBa_J23100, BBa_J23104, BBa_J23105, BBa_I14018, BBa_J45992, BBa_J23118, BBa_J23116, BBa_J23115, BBa_J23113, BBa_J23112, BBa_J23111, BBa_J23108, BBa_J23107, BBa_J23106, BBa_I14033, BBa_K256002, BBa_K1330002, BBa_J44002, BBa_J23150, BBa_I14034, Oxb19, oxb20, BBa_K088007, Ptet, Ptrc, PlacUV5, BBa_K292001, BBa_K292000, BBa_K137031 and BBa_K137029.

"Exogenous promoter" as used herein refers to a promoter that is operably linked to a coding region, wherein the promoter is not a promoter naturally associated with the coding region in the genome of the organism. A promoter in the genome that is naturally associated or linked to a coding region is called the "endogenous promoter" of the coding region.

The "exogenous environmental condition" or "environmental condition" mentioned in the present disclosure refers to the environment in which the promoter described herein is directly or indirectly induced, and refer to the environment or environmental condition outside the engineered microorganism, which are related to the in vitro culture conditions of the microorganism. "Exogenous environmental condition" can also refer to conditions during the growth, production and manufacturing of engineered microorganisms. Such conditions include aerobic culture conditions, anaerobic culture conditions, hypoxic culture conditions and other conditions at a set oxygen concentration. Such conditions also include the presence of chemical and/or nutritional inducers in the culture medium, such as tetracycline, arabinose, IPTG, rhamnose, etc. Such conditions further include the environmental temperature when the engineered microorganism is in the mammal.

"Intestine" as described in the present disclosure refers to the organs, glands, tracts and systems responsible for the transfer and digestion of food, absorption of nutrients, and excretion of waste products. In humans, the intestines include the gastrointestinal tract (GI), which begins in the mouth and ends in the anus, as well as the esophagus, stomach, small intestine, and large intestine. The intestines also include accessory organs and glands, such as the spleen, liver, gallbladder, and pancreas. The upper gastrointestinal tract consists of the esophagus, stomach and duodenum of the small intestine. The lower part of the gastrointestinal tract includes the remainder of the small intestine, namely the jejunum and ileum, and all of the large intestine, namely the cecum, colon, rectum, and anal canal. Bacteria can be found throughout the intestines, such as the gastrointestinal tract.

The "oxygen level-dependent promoter" or "oxygen level-dependent regulatory region" as described in the present disclosure refers to a nucleic acid sequence capable of binding by one or more oxygen level-sensing transcription factors, wherein the binding and/or activating factor of the corresponding transcription activates downstream gene expression.

"Non-pathogenic bacteria" as described in the present disclosure refers to bacteria that are unable to cause diseases or harmful responses in the host. Non-pathogenic bacteria can be commensal bacteria. Examples of non-pathogenic bacteria include, but are not limited to, *Bacillus, Bacteroides, Bifidobacterium, Brevibacteria, Clostridium, Enterococcus, Escherichia coli, Lactobacillus, Lactococcus, Saccharomyces* and *Staphylococcus, Bacillus coagulans, Bacillus subtilis, Bacteroides fragilis, Bacteroides subtili, Bacteroides thetaiotaomicron, Bifidobacterium bifidum, Bifidobacterium infantis, Bifidobacterium lactis, Bifidobacterium longum, Clostridium butyricum, Enterococcus faecium, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis* and *Saccharomyces boulardii.* Naturally pathogenic bacteria can be genetically engineered to provide reduced or eliminated pathogenicity.

"Probiotic" as described in the present disclosure refers to live, non-pathogenic microorganisms, such as bacteria, that are capable of conferring health benefits on host organisms containing appropriate amounts of the microorganisms. The host organism may be a mammal, in particular a human.

"Auxotrophy" as described in the present disclosure refers to a host cell (e.g., a microbial strain) that requires an external source of a specific metabolite for growth and the specific metabolite cannot be synthesized due to an acquired genetic defect. The term "auxotrophy-related gene" as used herein refers to genes required for the survival of host cells (e.g., microorganisms such as bacteria). The auxotrophy-related gene may be required for the production of nutrients necessary for the survival or growth of microorganisms, or may be required for detecting the signal of the activity of regulatory transcription factors in the environment, in which the loss of the signal will lead to cell death.

The terms "alleviation" and "treatment" and their synonyms as used herein refer to the amelioration of a disease, disorder and/or condition. "Alleviation" and "treatment" may be an improvement in at least one measurable physical parameter that is not necessarily identifiable by the patient. "Alleviation" and "treatment" may also refer to inhibiting the progression of a disease, disorder and/or condition physically (e.g., stabilizing identifiable symptoms), physiologically (e.g., stabilizing physical parameters), or both. "Alleviation" and "treatment" may also refer to slowing the progression of or reversal of a disease, disorder and/or condition.

As used herein, the term "treatment" is intended to encompass "prevention." "Prevention" and its synonyms as used herein refer to delaying the onset of a particular disease, disorder and/or condition or symptoms associated with such disease, disorder and/or condition, or reducing the risk of acquiring such disease, disorder and/or condition.

"Pharmaceutical composition" as described in the present disclosure refers to a preparation of the engineered microorganism or the engineered microorganism composition described in the present disclosure and other ingredients such as pharmaceutically acceptable carriers and/or excipients.

"Pharmaceutically acceptable carrier" as described in the present disclosure refers to the carrier or diluent or adjuvant which is used for the formulation or administration of engineered microorganisms or engineered microorganism compositions and is not a necessary active ingredient in itself and has no excessive toxicity after administration. Suitable pharmaceutically acceptable carriers are well known to those of ordinary skill in the art and include, but are not limited to, calcium bicarbonate, calcium phosphate, various sugar and starch types, cellulose derivatives, gelatin, vegetable oils, polyethylene glycol and surfactants, including, for example, polysorbate 20.

"Physiologically acceptable carrier" as described in the present disclosure refers to a carrier or diluent or adjuvant that does not cause significant irritation to the organism and does not eliminate the biological activity and properties of the administered engineered microorganism or engineered microorganism composition.

"Diseases and/or conditions associated with hyperphenylalaninemia" as described in the present disclosure include, but are not limited to: phenylketonuria (such as classic or typical phenylketonuria and atypical phenylketonuria), mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.

"Hyperphenylalaninemia" or "excess phenylalanine" as described in the present disclosure refers to an increased or abnormally high concentration of phenylalanine in the body. In some embodiments, the diagnostic signal for hyperphenylalaninemia is a blood phenylalanine level of at least 2 mg/dl, at least 4 mg/dl, at least 6 mg/dl, at least 8 mg/dl, at least 10 mg/dl, at least 12 mg/dl, at least 14 mg/dl, at least 16 mg/dl, at least 18 mg/dl, at least 20 mg/dl, or at least 25 mg/dl.

"Hyperphenylalaninemia" as described in the present disclosure includes, but is not limited to, phenylketonuria (such as classic or typical phenylketonuria and atypical phenylketonuria), permanent mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.

"Diseases and/or conditions associated to phenylalanine metabolism disorders" as described in the present disclosure are synonymous with "diseases and/or conditions associated to hyperphenylalaninemia", including: phenylketonuria (such as classic or typical phenylketonuria and atypical phenylketonuria), permanent mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.

### Engineered microorganisms

The engineered microorganism described in the present disclosure is capable of reducing excess phenylalanine. In some embodiments, the engineered microorganism is a bacterium or a yeast. In some embodiments, the engineered microorganism is a non-pathogenic bacterium. In some embodiments, the engineered microorganism is a commensal bacterium. In some embodiments, the engineered microorganism is a fungus. In some embodiments, the engineered microorganism is a probiotic. In some embodiments, the engineered microorganism is a naturally pathogenic bacterium that has been modified or mutated to reduce or eliminate pathogenicity. In some embodiments, the engineered microorganism is a Gram-negative bacterium. In some embodiments, the engineered microorganism is a Gram-positive bacterium. In some embodiments, the engineered microorganism is selected from the group comprising, but not limited to, *Bacteroides, Bifidobacterium, Clostridium, Escherichia, Lactobacillus* and *Lactococcus.*

In some embodiments, the engineered microorganism is *Escherichia coli.* In some embodiments, the engineered microorganism is *Escherichia coli* Nissle 1917 (*E. coli Nissle*), a gram-negative bacterium of *Enterobacteriaceae* which has evolved into one of the best characterized probiotics. This strain is characterized by being completely harmless and having GRAS (generally recognized as safe) status. Genome sequencing confirmed that *E. coli Nissle* lacks prominent virulence factors (e.g., *E. coli* α-hemolysin, P-fimbrial adhesin). In addition, E. *coli Nissle* has been shown not to carry pathogenic adhesion factors, not to produce any enterotoxins or cytotoxins, not to be invasive, and not to be uropathogenic. As early as 1917, E. *coli Nissle* was packaged as pharmaceutical capsules (called Mutaflor) for therapeutic use. Since then, *E. coli Nissle* has been used in vivo to treat ulcerative colitis in humans, used in vivo to treat inflammatory bowel disease, Crohn's disease, and pouchitis in humans, and used in vitro to inhibit enteroinvasive *Salmonella, Legionella, Yersinia,* and *Shigella.* It is generally accepted that the safety and utility of *E. coli Nissle* as a therapeutic vector have been convincingly demonstrated.

In some embodiments of the disclosure, the engineered microorganism is auxotrophic. In some embodiments of the present disclosure, the auxotrophy is any one or a combination of uracil, thymine, leucine, histidine, tryptophan, lysine, methionine, adenine, non-naturally occurring amino acid, and diaminoacrylic acid auxotrophys. In some embodiments of the present disclosure, the auxotrophy is a diaminoacrylic acid auxotrophy and/or a thymine auxotrophy. In some embodiments of the present disclosure, the auxotrophy is compensated when engineered microorganisms are present in the intestine in mammal. In some embodiments of the present disclosure, wherein the intestine in mammal is an intestine in human. In some embodiments, the engineered microorganism includes inactivation or deletion of at least one auxotrophy-related gene. In some embodiments, the auxotrophy-related gene is selected from: any one of or a combination of thyA, cysE, glnA, ilvD, leuB, lysA, serA, metA, glyA, hisB, ilvA, pheA, proA, thrC, trpC, tyrA, uraA, dapF, flhD, metB, metC, proAB, yhbV, yagG, hemB, secD, secF, ribD, ribE, thiL, dxs, ispA, dnaX, adk, hemH, IpxH, cysS, fold, rplT, infC, thrS, nadE, gapA, yeaZ, aspS, argS, pgsA, yeflA, metG, folE, yejM, gyrA, nrdA, nrdB, folC, accD, fabB, gltX, ligA, zipA, dapE, dapA, der, hisS, ispG, suhB, tadA, acpS, era, rnc, fisB, eno, pyrG, chpR, Igt, pgk, yqgD, metK, yqgF, plsC, ygiT, pare, ribB, cca, ygjD, tdcF, yraL, yihA, ftsN, murl, murB, birA, secE, nusG, rplJ, rplL, rpoB, rpoC, ubiA, plsB, lexA, dnaB, ssb, alsK, groS, psd, orn, yjeE, rpsR, chpS, ppa, valS, yjgP, yjgQ, dnaC, ribF, IspA, ispH, dapB, folA, imp, yabQ, flsL, flsl, murE, murF, mraY, murD, ftsW, murG, murC, ftsQ, ftsA, ftsZ, IpxC, secM, secA, can, folK, hemL, yadR, dapD, map, rpsB, in/B, nusA, ftsH, obgE, rpmA, rplU, ispB, murA, yrbB, yrbK, yhbN, rpsl, rplM, degS, mreD, mreC, mreB, accB, accC, yrdC, def, fint, rplQ, rpoA, rpsD, rpsK, rpsM, entD, mrdB, mrdA, nadD, hlepB, rpoE, pssA, yfiO, rplS, trmD, rpsP, ffh, grpE, yfjB, csrA, ispF, ispD, rplW, rplD, rplC, rpsJ, fusA, rpsG, rpsL, trpS, yr/F, asd, rpoH, ftsX, ftsE, ftsY, frr, dxr, ispU, rfaK, kdtA, coaD, rpmB, djp, dut, gmk, spot, gyrB, dnaN, dnaA, rpmH, rnpA, yidC, tnaB, glmS, glmU, wzyE, hemD, hemC, yigP, ubiB, ubiD, hemG, secY, rplO, rpmD, rpsE, rplR, rplF, rpsH, rpsN, rplE, rplX, rplN, rpsQ, rpmC, rplP, rpsC, rplV, rpsS, rplB, cdsA, yaeL, yaeT, lpxD, fabZ, IpxA, IpxB, dnaE, accA, tilS, proS, yafF, tsf, pyrH, olA, rlpB, leuS, Int, glnS, fldA, cydA, in/A, cydC, ftsK, lolA, serS, rpsA, msbA, IpxK, kdsB, mukF, mukE, mukB, asnS, fabA, mviN, rne, yceQ, fabD, fabG, acpP, tmk, holB, lolC, lolD, lolE, purB, ymflC, minE, mind, pth, rsA, ispE, lolB, hemA, prfA, prmC, kdsA, topA, ribA, fabi, racR, dicA, yd B, tyrS, ribC, ydiL, pheT, pheS, yhhQ, bcsB, glyQ, yibJ and gpsA.

In some embodiments of the present disclosure, the flagellar structure of the engineered microorganism has been modified to have functional defects or loss, so that the residence time of the engineered microorganism in the intestine is increased, which will be more conducive to the in vivo metabolism of phenylalanine. In some embodiments of the present disclosure, the modification of flagellar structure is achieved by mutation or deletion of part or all of flagellar synthesis-related genes. In some embodiments of the present disclosure, the engineered microorganism is Escherichia coli, and the flagellar synthesis-related genes are bscA and/or fliC genes.

### Enzymes and exogenous genes

According to an embodiment of the present disclosure, an engineered microorganism into which a exogenous gene is introduced can be provided, wherein the exogenous gene includes: one or more genes encoding an enzyme capable of converting phenylalanine to phenylpyruvate; one or more genes encoding an enzyme capable of converting phenylpyruvate to phenylacetaldehyde; one or more genes encoding an enzyme capable of converting phenylacetaldehyde to phenylethanol; and one or more genes encoding a protein capable of transporting phenylalanine into the engineered microorganism. In some embodiments of the present disclosure, the engineered microorganism is capable of metabolizing phenylalanine in the intestine in human and/or mammal. In some embodiments of the present disclosure, the exogenous gene is integrated into the genome or located on an expression plasmid.

In some embodiments of the present disclosure, the gene encoding an enzyme capable of converting phenylalanine to phenylpyruvate is selected from one or more of a gene encoding transaminase, a gene encoding dehydrogenase, a gene encoding deaminase, and functional equivalents thereof, and the functional equivalents retain at least part of the activities of the related enzymes.

In some embodiments of the present disclosure, the gene encoding dehydrogenase is a gene encoding phenylalanine dehydrogenase; in other embodiments, the enzyme capable of converting phenylalanine to phenylpyruvate includes phenylalanine dehydrogenase and an transaminase (e.g., aromatic amino acid transaminase). The present disclosure unexpectedly discovered that, at least in some embodiments, the use of phenylalanine dehydrogenase compared to transaminase can confer more metabolic advantages to engineered microorganisms to a certain extent. In some embodiments of the present disclosure, the gene encoding phenylalanine dehydrogenase is derived from a virus, fungus, and/or bacterium. In some embodiments of the present disclosure, the gene encoding phenylalanine dehydrogenase is derived from *Sporosarcina ureae* and/or *Bacillus,* such as *Sporosarcina ureae* SCRC-R04, *Lysinibacillus sphaericus* SCRC-R79a, *Bacillus badius,* and/or *Bacillus sp.* SLBN-3. In some embodiments of the present disclosure, the gene encoding phenylalanine dehydrogenase is selected from PheDH (Phenylalanine Dehydrogenase) of *Bacillus sp.* SLBN-3. In some embodiments of the present disclosure, the phenylalanine dehydrogenase has an amino acid sequence as shown in SEQ ID NO: 6; or the gene encoding the phenylalanine dehydrogenase has a nucleotide sequence as shown in SEQ ID NO: 17.

In some embodiments of the present disclosure, the gene encoding transaminase is a gene encoding aromatic amino acid transaminase In some embodiments of the present disclosure, the gene encoding aromatic amino acid transaminase is derived from a virus, fungus, and/or bacterium. In some embodiments of the present disclosure, the gene encoding aromatic amino acid transaminase is derived from E. coli and/or yeast, such as *Escherichia coli* BL21 (DE3) and/or *Saccharomyces cerevisiae* S288C. In some embodiments of the present disclosure, the gene encoding aromatic amino acid transaminase is selected from TyrB (Tyrosine aminotransferase) of *Escherichia coli* BL21(DE3) or ARO8 (Bifunctional 2-aminoadipate transaminase/aromatic-amino-acid: 2-oxoglutarate transaminase) of *Saccharomyces cerevisiae* S288C. In some embodiments of the present disclosure, the aromatic amino acid transaminase has an amino acid sequence as shown in SEQ ID NO: 1 or SEQ ID NO: 2; or the gene encoding the aromatic amino acid transaminase has a nucleotide sequence as shown in SEQ ID NO: 15 or SEQ ID NO: 90.

In some embodiments of the present disclosure, the engineered microorganism further comprises one or more of a gene encoding glutamate dehydrogenase and functional equivalents thereof, which functional equivalents retain at least part of the activity of the related enzyme, particularly when the engineered microorganism comprises a transaminase (e.g., an aromatic amino acid transaminase). Glutamate dehydrogenase can couple the transamination reaction of phenylalanine with the reduction reaction of phenylacetaldehyde, which realizes the regeneration of cofactors and provides more α-ketoglutarate for the transamination reaction, and further increases the rate of phenylalanine metabolism. In some embodiments of the present disclosure, the gene encoding glutamate dehydrogenase is derived from a virus, fungus, and/or bacterium. In some embodiments of the present disclosure, the gene encoding glutamate dehydrogenase is derived from yeast and/or *Clostridioides difficile,* such as *Saccharomyces cerevisiae* S288C and/or *Clostridioides difficile.* In some embodiments of the present disclosure, the gene encoding glutamate dehydrogenase is selected from GDH2 (Glutamate dehydrogenase (NAD+)) of *Saccharomyces cerevisiae* S288C or *Clostridioides difficile.* In some embodiments of the present disclosure, the glutamate dehydrogenase has an amino acid sequence as shown in SEQ ID NO: 11 or SEQ ID NO: 12; or the gene encoding the glutamate dehydrogenase has a nucleotide sequence as shown in SEQ ID NO: 20 or SEQ ID NO: 97.

In some embodiments of the present disclosure, the gene encoding deaminase is a gene encoding L-amino acid deaminase. In some embodiments of the present disclosure, the gene encoding L-amino acid deaminase is derived from a virus, fungus, and/or bacterium. In some embodiments of the present disclosure, the gene encoding L-amino acid deaminase is derived from *Escherichia coli,* yeast, and/or *Proteus mirabilis,* such as *Escherichia coli* BL21 (DE3), *Saccharomyces cerevisiae* S288C, and/or *Proteus mirabilis* HI4320. In some embodiments of the present disclosure, the gene encoding L-amino acid deaminase is selected from LAAD (L-amino acid deaminase) of *Escherichia coli* BL21 (DE3), *Saccharomyces cerevisiae* S288C, or *Proteus mirabilis* HI4320. In some embodiments of the present disclosure, the L-amino acid deaminase has an amino acid sequence as shown in SEQ ID NO: 89, or the gene encoding the L-amino acid deaminase has a nucleotide sequence as shown in SEQ ID NO: 91.

In some embodiments of the present disclosure, the gene encoding an enzyme capable of converting phenylalanine to phenylpyruvate includes at least one of the following group: (1) a gene encoding phenylalanine dehydrogenase; (2) a gene encoding aromatic amino acid transaminase; (3) a gene encoding aromatic amino acid transaminase and a gene encoding phenylalanine dehydrogenase; (4) a gene encoding aromatic amino acid transaminase and a gene encoding L-amino acid deaminase; (5) a gene encoding phenylalanine dehydrogenase and a gene encoding L-amino acid deaminase; or (6) a gene encoding aromatic amino acid transaminase, a gene encoding phenylalanine dehydrogenase, and a gene encoding L-amino acid deaminase.

In some embodiments of the present disclosure, the gene encoding an enzyme capable of converting phenylpyruvate to phenylacetaldehyde is selected from one or more of a gene encoding phenylpyruvate decarboxylase, a gene encoding α-keto acid decarboxylase, and functional equivalents thereof, and the functional equivalents retain at least part of the activities of the related enzymes. In some embodiments of the present disclosure, the gene encoding phenylpyruvate decarboxylase is derived from a virus, fungus, and/or bacterium. In some embodiments of the present disclosure, the gene encoding phenylpyruvate decarboxylase is derived from a yeast, such as *Saccharomyces cerevisiae* S288C. In some embodiments of the present disclosure, the gene encoding phenylpyruvate decarboxylase is ARO10 (Phenylpyruvate decarboxylase) of *Saccharomyces cerevisiae* S288C. In some embodiments of the present disclosure, the phenylpyruvate decarboxylase has an amino acid sequence as shown in SEQ ID NO: 7; or the gene encoding the phenylpyruvate decarboxylase has a nucleotide sequence as shown in SEQ ID NO: 18. In some embodiments of the present disclosure, the gene encoding α-keto acid decarboxylase is derived from a virus, fungus, and/or bacterium. In some embodiments of the present disclosure, the gene encoding α-keto acid decarboxylase is derived from a yeast and/or *Proteus mirabilis,* such as *Saccharomyces cerevisiae* S288C and/or *Proteus mirabilis* JN458. In some embodiments of the present disclosure, the gene encoding α-keto acid decarboxylase is selected from at least one of KDC (α-keto-acid decarboxylase) of *Saccharomyces cerevisiae* S288C or *Proteus mirabilis* JN458. In some embodiments of the present disclosure, the α-keto acid decarboxylase has a sequence as shown in SEQ ID NO: 8; or the gene encoding the α-keto acid decarboxylase has a nucleotide sequence as shown in SEQ ID NO: 95.

In some embodiments of the present disclosure, the gene encoding an enzyme capable of converting phenylacetaldehyde into phenylethanol is selected from one or more of a gene encoding aldehyde reductase and functional equivalents thereof, and the functional equivalents retain at least part of the activities of the related enzymes. In some embodiments of the present disclosure, the gene encoding aldehyde reductase is derived from a virus, fungus, and/or bacterium. In some embodiments of the present disclosure, the gene encoding aldehyde reductase is derived from *Escherichia coli* and/or *Lactobacillus brevis,* such as *Escherichia coli* str. K-12 substr. MG1655 and/or *Lactobacillus brevis.* In some embodiments of the present disclosure, the gene encoding aldehyde reductase is selected from YahK (NADPH-dependent aldehyde reductase) of *Escherichia coli* str. K-12 substr. MG1655 or ADH (Alcohol dehydrogenase) of *Lactobacillus brevis.* In some embodiments of the present disclosure, the aldehyde reductase has an amino acid sequence as shown in SEQ ID NO: 9 or SEQ ID NO: 10; or the gene encoding the aldehyde reductase has a nucleotide sequence as shown in SEQ ID NO: 19 or SEQ ID NO: 96.

In some embodiments of the present disclosure, the gene encoding a protein capable of transporting phenylalanine into the engineered microorganism is selected from one or more of a gene encoding phenylalanine transporter and functional equivalents thereof, and the functional equivalents retain at least part of the activities of the related enzymes. In some embodiments of the present disclosure, the gene encoding phenylalanine transporter is derived from a virus, fungus, and/or bacterium. In some embodiments of the present disclosure, the phenylalanine transporter is encoded by a phenylalanine transporter gene derived from bacteria including, but not limited to, *Acinetobacter calcoaceticus, Salmonella enteritidis,* and *Escherichia coli.* In some embodiments of the present disclosure, the gene encoding phenylalanine transporter is derived from *E. coli.* In some embodiments, the phenylalanine transporter is encoded by a PHEP gene derived from bacteria. In some embodiments, the phenylalanine transporter is encoded by a AROP gene derived from bacteria. In some embodiments, the phenylalanine transporter is encoded by the bacterially derived LIV-binding protein and LS-binding protein and LIVHMGF genes. In some embodiments, the engineered microorganism contains at least one type of phenylalanine transporter selected from PHEP, AROP and LIV-I/LS. In some embodiments of the present disclosure, the gene encoding phenylalanine transporter is PheP (Phenylalanine: H(+) symporter) of *E. coli.* In some embodiments of the present disclosure, the phenylalanine transporter has an amino acid sequence as shown in SEQ ID NO: 13; or the gene encoding the phenylalanine transporter has a nucleotide sequence as shown in SEQ ID NO: 21.

In some embodiments of the present disclosure, any one, two, three, four, five, six, a plurality of or all of the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, the gene encoding glutamate dehydrogenase, and/or the gene encoding phenylalanine transporter are optionally operably linked to one or more of identical or different promoters. In some embodiments of the present disclosure, the gene encoding aromatic amino acid transaminase and the gene encoding glutamate dehydrogenase share the same promoter. In some embodiments of the present disclosure, the gene encoding phenylpyruvate decarboxylase and the gene encoding aldehyde reductase share the same promoter. In some embodiments of the present disclosure, the gene encoding phenylalanine dehydrogenase and the gene encoding phenylalanine transporter share the same promoter. In some embodiments of the present disclosure, the gene encoding L-amino acid deaminase alone is operably linked to a promoter. In some embodiments of the present disclosure, the gene encoding phenylalanine transporter alone is operably linked to a promoter. In some embodiments of the present disclosure, the promoter is an endogenous promoter or an exogenous promoter. In some embodiments of the present disclosure, the promoter is an inducible promoter or a constitutive promoter. In some embodiments of the present disclosure, the promoter is a directly or indirectly inducible promoter. In some embodiments, the promoter is a pH-dependent promoter. In some embodiments, the promoter is an oxygen level-dependent promoter. In some embodiments of the present disclosure, the promoter is induced directly or indirectly by an exogenous environmental condition. In some embodiments of the present disclosure, the exogenous environmental condition refers to exogenous environmental conditions in the mammal intestine. In some embodiments of the present disclosure, the mammal intestine refers to an intestine in human. In some embodiments, the exogenous environmental condition refers to the upper gastrointestinal tract of a mammal. In some embodiments, the exogenous environmental condition refers to the lower gastrointestinal tract of a mammal. In some embodiments, the exogenous environmental condition refers to the small intestine of a mammal. In some embodiments, the exogenous environmental condition refers to a hypoxic condition, a microaerophilic condition, or an anaerobic condition, such as the environment in the intestine in mammal. In some embodiments, the exogenous environmental condition refers to molecules or metabolites present in the intestine of a mammal in a healthy or disease state. In some embodiments, the exogenous environmental condition is a tissue-specific or disease-specific metabolite or molecule. In some embodiments, the exogenous environmental condition is a low pH environment. In some embodiments, the promoter is selected from at least one of PfnrS, FDHF, Ptet, Pbba, Ptrc, Pvan, or PBAD. In some embodiments of the present disclosure, the sequence of the PfnrS promoter is as shown in SEQ ID NO. 22. In some embodiments of the present disclosure, the sequence of the FDHF promoter is as shown in SEQ ID NO. 24. In some embodiments of the present disclosure, the sequence of the Ptet promoter is as shown in SEQ ID NO. 100. In some embodiments of the present disclosure, the sequence of the Pbba promoter is as shown in SEQ ID NO. 98. In some embodiments of the present disclosure, the sequence of the Ptrc promoter is as shown in SEQ ID NO. 99. In some embodiments of the present disclosure, the Pvan promoter is a vanillic acid-induced promoter, and has a sequence as shown in SEQ ID NO: 101. In some embodiments of the present disclosure, the sequence of the PBAD promoter is as shown in SEQ ID NO: 102. It has been known that the activity of the vanillic acid promoter can be inhibited by the suppressor expression cassette VanRAM (the coding sequence of which as set forth in SEQ ID NO: 111), and that said inhibition can be relieved by adding vanillic acid, thereby allowing the vanillic acid promoter to exhibit promoter activity. Through this mechanism, the inducible regulation of the promoter can be achieved, and when VanRAM is not present endogenously in the engineered microorganism, the vanillic acid promoter is in a state of continuously activation, at which point additional VanRAM needs to be added in order to achieve inducible expression. Therefore, in some embodiments of the present disclosure, when the promoter is a Pvan promoter, an expression cassette capable of expressing one or more copies of the vanillic acid suppressor VanRAM can also be simultaneously transformed into the genome of the engineered microorganism.

In some embodiments of the present disclosure, the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, the gene encoding glutamate dehydrogenase, and/or the gene encoding phenylalanine transporter is located within one or more expression cassettes. In some embodiments of the present disclosure, the expression cassette is present in the form of single copy, two copies, three copies, four copies or more copies. In some embodiments, the expression cassette is located on a plasmid. In some embodiments of the present disclosure, the expression cassette is stably integrated in one, two, or more identical or different genomic sites of the engineered microorganism. In some embodiments of the present disclosure, the genomic site is selected from at least one of yicS site, malPT site, malE site, exo site, rhtB/C site, agaI/rsml site, araBD site, yghx site, ldhA site, araAB site, lacZ site, kefB site, maeB site, nth/tppB site, and/or tkrA site.

In some embodiments of the present disclosure, the engineered microorganism of the present disclosure includes a first expression cassette and a second expression cassette, wherein the first expression cassette includes a first promoter, and the gene encoding aromatic amino acid transaminase and the gene encoding glutamate dehydrogenase operably linked to the first promoter; the second expression cassette includes a second promoter, and the gene encoding phenylpyruvate decarboxylase and the gene encoding aldehyde reductase operably linked to the second promoter; the first promoter or the second promoter may be identical or different. In some embodiments, the engineered microorganism further includes a third expression cassette, the third expression cassette includes the gene encoding phenylalanine transporter operably linked to the third promoter, and the third promoter may be identical to the first promoter and/or the second promoter, or may be different from both the first promoter and the second promoter. In some embodiments of the present disclosure, the first expression cassette is present in the form of single copy or two copies. In some embodiments of the present disclosure, the second expression cassette is present in the form of single copy, two copies, three copies, or four copies. In some embodiments of the present disclosure, the third expression cassette is present in the form of single copy. In some embodiments of the present disclosure, the first expression cassette is integrated at the yghx site and/or the araAB site. In some embodiments of the present disclosure, the second expression cassette is integrated at the ldhA site, lacZ site, yghx site, yjcS site and/or agaI/rsml site. In some embodiments of the present disclosure, the third expression cassette is integrated at the kefB site.

In some embodiments of the present disclosure, the engineered microorganism of the present disclosure includes a fourth expression cassette and a second expression cassette, wherein the fourth expression cassette includes a fourth promoter, and the gene encoding phenylalanine dehydrogenase and the gene encoding phenylalanine transporter operably linked to the fourth promoter; the second expression cassette includes a second promoter, and the gene encoding phenylpyruvate decarboxylase and the gene encoding aldehyde reductase operably linked to the second promoter; the fourth promoter or the second promoter may be identical or different. In some embodiments, the engineered microorganism further includes a first expression cassette, the first expression cassette includes a first promoter, the gene encoding aromatic amino acid transaminase and the gene encoding glutamate dehydrogenase operably linked to the first promoter; the first promoter may be the identical to the fourth promoter and/or the second promoter, or may be different from both the fourth promoter and the second promoter. In some embodiments, the engineered microorganism further includes a fifth expression cassette, the fifth expression cassette includes a fifth promoter, the gene encoding L-amino acid deaminase operably linked to the fifth promoter; the fifth promoter is identical to or different from the fourth promoter, the second promoter and/or the first promoter. In some embodiments of the present disclosure, the expression cassette is present in the form of single copy, two copies, three copies, four copies or more copies. In some embodiments of the present disclosure, the fourth expression cassette is present in the form of single copy or two copies. In some embodiments of the present disclosure, the second expression cassette is present in the form of single copy, two copies, three copies, or four copies. In some embodiments of the present disclosure, the first expression cassette is present in the form of single copy or two copies. In some embodiments of the present disclosure, the fifth expression cassette is present in the form of single copy. In some embodiments of the present disclosure, the fourth expression cassette is integrated at the kefB site, ldhA site, nth/tppB site, maeB site and/or tkrA site. In some embodiments of the present disclosure, the second expression cassette is integrated at the ldhA site, yghx site, lacZ site, yjcS site and/or agaI/rsml site. In some embodiments of the present disclosure, the first expression cassette is integrated at the yghX site and/or araAB site. In some embodiments of the present disclosure, the fifth expression cassette is integrated at the rhtB/C site.

In some embodiments of the present disclosure, all or part of the first promoter, second promoter, third promoter, and fourth promoter are selected from at least one of PfnrS (sequence as shown in any one of SEQ ID NO: 22-23) and/or Pvan (sequence as shown in SEQ ID NO: 101), and the fifth promoter is PBAD (sequence as shown in SEQ ID NO: 102).

In some embodiments of the present disclosure, the engineered microorganism of the present disclosure includes single copy of the fourth expression cassette, and the fourth expression cassette is integrated at the kefB site; two copies of the second expression cassette, wherein the second expression cassette is integrated at the ldhA site and the lacZ site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the PfnrS promoter (sequence as shown in SEQ ID NO: 22), and the promoter used in the fifth expression cassette is the PBAD promoter (sequence as shown in SEQ ID NO: 101).

In some embodiments of the present disclosure, the engineered microorganism of the present disclosure includes single copy of the fourth expression cassette, and the fourth expression cassette is integrated at the nth/tppB site; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the Pvan promoter (sequence as shown in SEQ ID NO: 101), and the promoter used in the fifth expression cassette is the PBAD promoter (sequence as shown in SEQ ID NO: 102).

In some embodiments of the present disclosure, the engineered microorganism of the present disclosure includes double copies of the fourth expression cassette, and the fourth expression cassette is integrated at the nth/tppB site and the tkrA site respectively; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the Pvan promoter (sequence as shown in SEQ ID NO: 101), and the promoter used in the fifth expression cassette is the PBAD promoter (sequence as shown in SEQ ID NO: 102).

In some embodiments of the present disclosure, the engineered microorganism of the present disclosure includes single copy of the fourth expression cassette, and the fourth expression cassette is integrated at the maeB site; three copies of the second expression cassette, wherein the second expression cassette is integrated at the yjcS site, the lacZ site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the single copy of the fourth expression cassette uses the PfnrS promoter, the three copies of the second expression cassette use the PfnrS promoter (sequence as shown in any one of SEQ ID NO: 22), the PfnrS promoter (sequence as shown in SEQ ID NO: 22) and the Pvan promoter (sequence as shown in SEQ ID NO: 101) respectively, the two copies of the first expression cassette use the PfnrS promoter (sequence as shown in SEQ ID NO: 22), and the Pvan promoter (sequence as shown in SEQ ID NO: 101) respectively, and the promoter used in the fifth expression cassette is the PBAD promoter (sequence as shown in SEQ ID NO: 102).

In some embodiments of the present disclosure, the engineered microorganism of the present disclosure includes two copies of the fourth expression cassette, and the fourth expression cassette is integrated at the maeB site and the kefB site; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the two copies of the fourth expression cassette use the PfnrS promoter, the three copies of the second expression cassette use the Pvan promoter, the Pvan promoter and the PfnrS promoter (sequence as shown in SEQ ID NO: 22) respectively, two copies of the first expression cassette use the Pvan promoter (sequence as shown in SEQ ID NO: 101), and the promoter used in the fifth expression cassette is the PBAD promoter (sequence as shown in SEQ ID NO: 102).

In some embodiments of the present disclosure, the engineered microorganism of the present disclosure includes single copy of the fourth expression cassette, and the fourth expression cassette is integrated at the kefB site; four copies of the second expression cassette, wherein the second expression cassette is integrated at the ldhA site and the lacZ site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the PfnrS promoter (sequence as shown in SEQ ID NO: 22), and the promoter used in the fifth expression cassette is the PBAD promoter (sequence as shown in SEQ ID NO: 102).

In some embodiments of the present disclosure, the engineered microorganism of the present disclosure includes two copies of the fourth expression cassette, and the fourth expression cassette is integrated at the ldhA site and the maeB site; two copies of the second expression cassette, wherein the second expression cassette is integrated at the yghX site and the lacZ site respectively; and single copy of the fifth expression cassette, wherein the fifth expression cassette is integrated at the rhtB/C site; among them, the promoter used in the fourth and second expression cassettes is the PfnrS promoter (sequence as shown in SEQ ID NO: 22), and the promoter used in the fifth expression cassette is the PBAD promoter (sequence as shown in SEQ ID NO: 102).

In some embodiments of the present disclosure, the engineered microorganism of the present disclosure includes single copy of the fourth expression cassette, and the fourth expression cassette is integrated at the ldhA site; and single copy of the second expression cassette, wherein the second expression cassette is integrated at the yghX site; among them, the promoter used in the fourth and second expression cassettes is the PfnrS promoter (sequence as shown in SEQ ID NO: 22).

In some embodiments of the present disclosure, the engineered microorganism of the present disclosure includes single copy of the first expression cassette, and the first expression cassette is integrated at the yghX site; single copy of the second expression cassette, wherein the second expression cassette is integrated at the ldhA site; and single copy of the third expression cassette, wherein the third expression cassette is integrated at the kefB site; among them, the promoter used in the first, second and third expression cassettes is the PfnrS promoter (sequence as shown in SEQ ID NO: 22).

In some embodiments of the present disclosure, the engineered microorganism of the present disclosure includes single copy of the first expression cassette, and the first expression cassette is integrated at the yghX site; and single copy of the second expression cassette, wherein the second expression cassette is integrated at the ldhA site; among them, the promoter used in the first and second expression cassettes is the PfnrS promoter (sequence as shown in SEQ ID NO: 22).

In some embodiments of the present disclosure, the engineered microorganism of the present disclosure includes two copies of the first expression cassette, and the first expression cassette is integrated at the yghX site and the araAB site respectively; and two copies of the second expression cassette, wherein the second expression cassette is integrated at the ldhA site and the lacZ site; among them, the promoter used in the first and second expression cassettes is the PfnrS promoter (sequence as shown in SEQ ID NO: 22).

In some embodiments of the present disclosure, the engineered microorganism of the present disclosure includes two copies of the first expression cassette, and the first expression cassette is integrated at the yghX site and the araAB site respectively; two copies of the second expression cassette, wherein the second expression cassette is integrated at the ldhA site and the lacZ site respectively; and single copy of the third expression cassette, wherein the third expression cassette is integrated at the kefB site; among them, the promoter used in the first, second and third expression cassettes is the PfnrS promoter (sequence as shown in SEQ ID NO: 22).

### Compositions and kits

According to one embodiment of the present disclosure, an engineered microorganism composition is provided, which includes more than one engineered microorganism, each of the more than one engineered microorganism independently includes any one or more of the following genes: a gene encoding an enzyme capable of converting phenylalanine to phenylpyruvate; a gene encoding an enzyme capable of converting phenylpyruvate to phenylacetaldehyde; a gene encoding an enzyme capable of converting phenylacetaldehyde to phenylethanol; and a gene encoding a protein capable of transporting phenylalanine into the engineered microorganism; wherein the engineered microorganism composition includes the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol and the gene encoding the protein capable of transporting phenylalanine into the engineered microorganism. In some embodiments of the present disclosure, each of the more than one engineered microorganism independently includes any one or more of the following genes: a gene encoding an enzyme capable of converting phenylalanine to phenylpyruvate; a gene encoding an enzyme capable of converting phenylpyruvate to phenylacetaldehyde; a gene encoding an enzyme capable of converting phenylacetaldehyde to phenylethanol; a gene encoding glutamate dehydrogenase; and a gene encoding a protein capable of transporting phenylalanine into the engineered microorganism; wherein the engineered microorganism composition includes the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenyl ethanol, the gene encoding glutamate dehydrogenase and the gene encoding the protein capable of transporting phenylalanine into the engineered microorganism.

According to one embodiment of the present disclosure, a composition is provided, the composition includes the engineered microorganism or the engineered microorganism composition according to the present disclosure and a pharmaceutically, nutritionally or physiologically acceptable carrier. In some embodiments of the present disclosure, the composition is a pharmaceutical composition useful for treating, alleviating, managing, ameliorating and/or preventing diseases and/or conditions associated with hyperphenylalaninemia.

The engineered microorganism may be present in the composition in an amount ranging from about 10⁴ to about 10¹³ colony forming units (CFU). For example, the effective amount of the engineered microorganism can be about 10⁵ CFU to about 10¹³ CFU, preferably about 10⁶ CFU to about 10¹³ CFU, preferably about 10⁷ CFU to about 10¹² CFU, more preferably about 10⁸ CFU to about 10¹² CFU. Engineered microorganisms can be living cells or can be dead cells.

In some embodiments of the present disclosure, the composition is an edible composition. In some embodiments of the present disclosure, the composition is a probiotic composition. In some embodiments of the present disclosure, the composition is a food supplement. In some embodiments, the compositions disclosed herein may be formulated for oral administration and may be a nutritional or nourishing composition, such as a food, a food supplement, a feed, or a feed supplement, for example a dairy product, such as a fermented dairy product, such as yogurt or yogurt drinks. In this case, the composition may include a nutritionally acceptable carrier, which may be a suitable food base. Those skilled in the art are aware of a variety of formulations that may encompass live or dead microorganisms and may be presented as food supplements (e.g., pills, tablets, etc.) or functional foods (e.g., beverages, fermented yogurt, etc.). The composition disclosed herein may also be formulated as a pharmaceutical agent in capsules, pills, liquid solutions, for example, formulated as encapsulated freeze-dried bacteria, and the like. In some embodiments, the composition is a probiotic composition.

The compositions disclosed herein may be formulated for a single administration or for multiple administrations to be effective for a given individual. For example, a single administration is effective to substantially reduce monitored symptoms of the targeted disease condition in a mammal subject administered the composition.

In some embodiments, the composition is formulated such that a single oral dose contains at least about 1 × 10⁴, 1 × 10⁵, 1 × 10⁶, 1 × 10⁷, 1 × 10⁸, 1 × 10⁹, 1 × 10¹⁰, 1 × 10¹¹, 1 × 10¹², 1 × 10¹³ CFU of engineered microorganisms (e.g., bacterial entities and/or fungal entities). In some embodiments, the composition includes 1 × 10⁸ - 1 × 10¹² CFU of the engineered microorganism of the present invention.

In some formulations, the composition includes at least about 0.5%, 1%, 2%, 5%, 10%, 20%, 30%, 40%, 50%, 60%, 70%, 80%, 90% or greater than 90% of the microorganisms of the present disclosure. In some formulations, the dose administered does not exceed 200, 300, 400, 500, 600, 700, 800, 900 milligrams or 1, 1.1, 1.2, 1.3, 1.4, 1.5, 1.6, 1.7, 1.8 or 1.9 grams of the engineered microorganism provided in the present application.

Physiologically acceptable carriers used for the compositions disclosed herein may include, for example, physiologically acceptable liquid, gel or solid carriers, aqueous vehicles, non-aqueous vehicles, antimicrobial agents, isotonic agents, buffers, antioxidants, suspending/dispersing agents, sequestering/chelating agents, diluents, adjuvants, excipients or non-toxic auxiliary substances, other components known in the art or various combinations thereof. In some embodiments, the composition described in the present disclosure is a liquid formulation, a solid formulation, or a semi-solid formulation. In some embodiments, the liquid formulation is selected from the group of: a solution product or a suspension product.

For further explanation, the aqueous vehicles may include, for example, sodium chloride injection, Ringer's injection, isotonic dextrose injection, sterile water injection, or dextrose and lactate Ringer's injection; the non-aqueous vehicles may include, for example, fixed oils of vegetable origin, cottonseed oil, corn oil, sesame oil, or peanut oil; The antimicrobial agents may be at bacteriostatic or fungistatic concentrations and/or may be added to a composition in a multi-dose container containing phenol or cresol, mercury, benzyl alcohol, chlorobutanol, methyl and propyl paraben, thimerosal, benzalkonium chloride and benzethonium chloride. The isotonic agents may include, for example, sodium chloride or dextrose; the buffers may include, for example, phosphate or citrate buffers; the antioxidants may include, for example, sodium bisulfate; the suspending agent and the dispersing agents may include, for example, sodium carboxymethylcellulose, hydroxypropylmethylcellulose or polyvinylpyrrolidone; the chelating agents may include, for example, ethylenediaminetetraacetic acid (EDTA) or ethylene glycol tetraacetic acid (EGTA), ethanol, polyethylene glycol, propylene glycol, sodium hydroxide, hydrochloric acid, citric acid or lactic acid. The suitable excipients may include, for example, water, physiological saline, dextrose, glycerol or ethanol. The suitable non-toxic auxiliary substances may include, for example, wetting agents or emulsifying agents, pH buffers, stabilizers, solubility enhancers or reagents such as sodium acetate, sorbitan monolaurate, triethanolamine oleate or cyclodextrins.

In some embodiments, the compositions provided herein can be pharmaceutical compositions. In some embodiments, the compositions provided herein can be food supplements. The compositions provided herein may include pharmaceutically, nutritionally/alimentarily, or physiologically acceptable carriers in addition to the genetically modified engineered microorganisms provided herein. The preferred form will depend on the intended mode of administration and (therapeutic) application. The carrier can be any compatible physiologically acceptable nontoxic substance suitable for delivering the genetically modified engineered microorganisms provided herein to the gastrointestinal tract of mammals (such as humans), preferably near or in the intestinal mucosal barrier (more preferably the colonic mucosal barrier) of mammals. In some embodiments, the dosage form of the pharmaceutical composition is selected from the group of: powders, pulvises, tablets, dragees, capsules, granules, suspensions, solutions, syrups, drops, sublingual tablets, or combinations thereof.

The composition may be a liquid solution, a suspension, an emulsion, a pill, a capsule, a tablet, a sustained release preparation or a pulvis. Oral preparations may contain standard carriers such as pharmaceutical grade mannitol, lactose, starch, magnesium stearate, polyvinylpyrrolidone, sodium saccharin, cellulose, magnesium carbonate, and the like.

The compositions of the present disclosure may be formulated in a conventional manner using one or more physiologically acceptable carriers, including excipients and auxiliaries, which facilitate processing of the active ingredients into compositions for pharmaceutical use. In some embodiments, the pharmaceutical composition is subjected to tableting, lyophilization, direct compression, conventional mixing, dissolution, granulation, grinding, emulsification, encapsulation, embedding, or spray drying to form tablets, granules, nanoparticles, nanocapsules, microcapsules, microtablets, pills, or powders, which may be enteric-coated or uncoated. An appropriate preparation depends on its route of administration.

The engineered microorganisms described in the present disclosure can be formulated as pharmaceutical compositions in any suitable dosage form (e.g., liquid, capsule, sachet, hard capsule, soft capsule, tablet, enteric-coated tablet, suspension powder, granule or matrix sustained release formulation for oral administration) and for any suitable type of administration (e.g., oral, immediate release, pulsatile release, delayed release or sustained release). The composition may be administered once or more daily, weekly or monthly. Engineering microorganisms can be formulated into pharmaceutical compositions containing one or more pharmaceutically acceptable carriers, thickeners, diluents, buffers, surfactants, neutral or cationic lipids, lipid complexes, liposomes, penetration enhancers, carrier compounds and other pharmaceutically acceptable carriers or agents.

The engineered microorganisms described in the present disclosure can be administered orally and formulated as tablets, pills, dragees, capsules, liquids, gels, syrups, slurries, suspensions, and the like. Pharmaceutical compositions for oral use may be manufactured using solid excipients, optionally by grinding the resulting mixture, and, if necessary, after adding suitable auxiliaries, processing the mixture of granules to obtain tablets or dragee cores. Suitable excipients include, but are not limited to, fillers such as sugars, including lactose, sucrose, mannitol or sorbitol; cellulose compositions such as corn starch, wheat starch, rice starch, potato starch, gelatin, tragacanth, methyl cellulose, hydroxypropyl methyl cellulose, sodium carboxymethylcellulose; and/or physiologically acceptable polymers such as polyvinylpyrrolidone (PVP) or polyethylene glycol (PEG). Disintegrants such as cross-linked polyvinylpyrrolidone, agar, alginic acid or salts thereof such as sodium alginate may also be added.

Tablets or capsules may be prepared by conventional methods with pharmaceutically acceptable excipients such as binding agents (e.g., pregelatinized corn starch, polyvinylpyrrolidone, hydroxypropyl methyl cellulose, carboxymethyl cellulose, polyethylene glycol, sucrose, glucose, sorbitol, starch, gums, kaolin and tragacanth); fillers (e.g., lactose, microcrystalline cellulose, or dibasic calcium phosphate); lubricants (e.g., calcium, aluminum, zinc, stearic acid, polyethylene glycol, sodium lauryl sulfate, starch, sodium benzoate, L-leucine, magnesium stearate, talc, or silicon dioxide); disintegrants (e.g., starch, potato starch, sodium starch glycolate, sugars, cellulose derivatives, silica powder); or wetting agents (e.g., sodium lauryl sulfate). Tablets may be coated by methods well known in the art. Coating shells may be present and common films include, but are not limited to, polylactide, polyglycolic acid, polyanhydride, other biodegradable polymers, alginate-polylysine-alginate (APA), alginate-polymethylene-co-guanidine-alginate (A-PMCG-A), hydroxymethyl acrylate-methyl methacrylate (HEMA-MMA), multi-layer HEMA-MMA-MAA, polyacrylonitrilevinylchloride (PAN-PVC), acrylonitrile/sodium methallylsulfonate (AN-69), polyethylene glycol/polypentamethylcyclopentasiloxane/polydimethylsiloxane (PEG/PD5/PDMS), poly N,N-dimethylacrylamide (PDMAAm), siliceous encapsulants, cellulose sulfate/sodium alginate/polymethylene-co-guanidine (CS/A/PMCG), cellulose acetate phthalate, calcium alginate, k-carrageenan-locust bean gum gel beads, gellan-xanthan gum beads, poly(lactide-co-glycolide), carrageenan, starch polyanhydride, starch polymethacrylate, polyamino acids and enteric coating polymers.

Liquid preparations for oral administration may take the form of solutions, syrups, suspensions, or dry products reconstituted with water or other suitable vehicles before use. Such liquid preparations may be prepared by conventional methods together with pharmaceutically acceptable agents: the pharmaceutically acceptable agents are such as suspending agents (for example, sorbitol syrup, cellulose derivatives or hydrogenated edible fat); emulsifiers (e.g., lecithin or gum arabic); non-aqueous vehicles (e.g., almond oil, oily esters, ethanol, or fractionated vegetable oils); and preservatives (for example, methyl or propyl paraben or sorbic acid). The preparations may also contain buffer salts, flavoring agents, coloring agents and sweetening agents, as appropriate. Preparations for oral administration may be suitably formulated for slow release, controlled release, or sustained release of the engineered microorganisms described in the present disclosure.

In some embodiments, the engineered microorganisms described in the present disclosure can be administered orally, for example, with an inert diluent or an assimilable edible carrier. The compounds may also be enclosed in hard- or soft-shell gelatin capsules, compressed into tablets, or incorporated directly into the subject's diet. For oral therapeutic administration, the compounds can be mixed with excipients and used in the form of ingestible tablets, buccal tablets, lozenges, capsules, elixirs, suspensions, syrups, wafers, and the like. In order to administer the compound of the present disclosure by means other than parenteral administration, it may be necessary to coat the compound with a material or to co-administer the compound with a material to prevent its inactivation.

In some embodiments, the composition is formulated for enteral administration, jejunal administration, duodenal administration, ileal administration, gastric shunt administration or intracolonic administration via nanoparticles, nanocapsules, microcapsules or microtablets (which are enteric coated or uncoated). Pharmaceutical compositions of the present disclosure may also be formulated as rectal compositions such as suppositories or retention enemas using, for example, conventional suppository bases such as cocoa butter or other glycerides. The compositions may be a suspension, solution or emulsion in oily or aqueous vehicles, and may contain suspending, stabilizing and/or dispersing agents.

In some embodiments, the present disclosure provides pharmaceutically acceptable compositions in a single dosage form. Single dosage forms may be in liquid or solid form. Single dosage forms can be administered directly to the patient without modification, or can be diluted or reconstituted prior to administration. In certain embodiments, a single dosage form may be administered as a bolus, such as a single injection, a single oral dose, including oral doses containing a plurality of tablets, capsules, pills, and the like. In alternative embodiments, a single dosage form may be administered over a period of time, such as by infusion.

Single dosage forms of the pharmaceutical compositions of the present disclosure can be prepared by: dispensing pharmaceutical compositions into smaller aliquots, into single-dose containers, into single-dose liquid forms, or single-dose solid forms, such as tablets, granules, nanoparticles, nanocapsules, microcapsules, microtablets, pellets or powders, which may be enteric-coated or uncoated. A single dose in solid form can be reconstituted by the addition of a liquid, usually sterile water or saline solution, before administration to the patient.

Dosage regimens can be adjusted to provide a therapeutic response. For example, a single bolus may be administered at one time, several divided doses may be administered over a predetermined period of time, or the dose may be decreased or increased as indicated by the treatment situation. Dose specifications are determined by the unique characteristics of the active compound and the specific therapeutic effect to be achieved. Dose values may vary with the type and severity of the condition to be alleviated. For any particular subject, the specific dosage regimen may be adjusted over time based on the individual needs and the professional judgment of the treating clinician.

In another embodiment, the composition can be delivered in a controlled release or sustained release system. In one embodiment, a pump may be used to achieve controlled or sustained release. In another embodiment, polymeric materials may be used to achieve controlled or sustained release of therapies of the present disclosure. Examples of polymers used in sustained release preparations include, but are not limited to, poly(2-hydroxyethyl methacrylate), poly(methyl methacrylate), poly(acrylic acid), poly(ethylene-co-vinyl acetate), poly(methacrylic acid), polyglycolide (PLG), polyanhydride, poly(N-vinylpyrrolidone), poly(vinyl alcohol), polyacrylamide, poly(ethylene glycol), polylactide (PLA), poly(lactide-co-glycolide) (PLGA) and polyorthoesters. Polymers used in sustained release preparations can be inert, free of leachable impurities, stable on storage, sterile and biodegradable. In some embodiments, controlled release systems or sustained release systems can be placed proximate the prophylactic or therapeutic target, thus requiring only a portion of the systemic dose. Any suitable technique known to those skilled in the art may be used.

Dose can depend on several factors, including severity and responsiveness of the disease, route of administration, duration of treatment (days to months to years), and time to improvement of disease.

In another aspect, the present disclosure provides a kit including the engineered microorganism of the present disclosure or the engineered microorganism composition of the present disclosure or the composition of the present disclosure.

If necessary, the kit may further include one or more of various conventional pharmaceutical kit components, such as a container with one or more pharmaceutically acceptable carriers, additional containers, etc., as will be obvious to those skilled in the art. Instructions may also be included in the kit as an insert or label indicating the amounts of components to be administered, guides for administration, and/or guides for mixing the components.

### Treatment methods and uses

The engineered microorganism described in the present disclosure includes one or more of a gene encoding an enzyme capable of converting phenylalanine to phenylpyruvate, a gene encoding an enzyme capable of converting phenylpyruvate to phenylacetaldehyde, a gene encoding an enzyme capable of converting phenylacetaldehyde into phenylethanol, a gene encoding glutamate dehydrogenase, and/or a gene encoding phenylalanine transporter, and can alleviate and/or treat diseases and/or conditions associated with hyperphenylalaninemia.

The engineered microorganism constructed in the present disclosure can effectively degrade phenylalanine and/or phenylpyruvate. Experiments have proven that the engineered microorganism effectively degraded phenylalanine and phenylalanine metabolites in Pah^{R408}W mice with phenylketonuria, showing good application prospects.

The present disclosure provides a method for alleviating and/or treating diseases and/or conditions associated with hyperphenylalaninemia, the method includes administering the engineered microorganism according to the present disclosure or the engineered microorganism composition according to the present disclosure or the composition according to the present disclosure or the kit according to the present disclosure to a patient in need thereof.

According to one embodiment of the present disclosure, the use of the engineered microorganism of the present disclosure or the engineered microorganism composition of the present disclosure in the preparation of a drug or a health product for alleviating and/or treating diseases and/or conditions associated with hyperphenylalaninemia can be provided.

In some embodiments of the present disclosure, the diseases and/or conditions associated with hyperphenylalaninemia include, but are not limited to: phenylketonuria (such as classic or typical phenylketonuria and atypical phenylketonuria), permanent mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.

### EXAMPLE

Hereinafter, examples of the present application will be described. The examples described below are illustrative and are only used to explain the present application and are not to be construed as limitations of the present application. If specific techniques or conditions are not specified in the examples, techniques or conditions described in the literature in the art or techniques or conditions according to the product instructions are followed. The reagents or instruments used therein for which manufacturers are not specified are all conventional products that are commercially available.

**Table 1: Detailed sequence information of enzymes, promoters and expression cassettes used in the examples**

| **Name** | **Abbreviation** | **Sequence type** | **Sequence** |
|---|---|---|---|
| Aromatic amino acid transaminase | TyrB | Amino acid | SEQ ID NO. 1 |
| Aromatic amino acid transaminase | ARO8 | Amino acid | SEQ ID NO.2 |
| L-amino acid deaminase | LAAD | Amino acid | SEQ ID NO.89 |
| Glutamate dehydrogenase | ScGDH2 | Amino acid | SEQ ID NO.11 |
| Glutamate dehydrogenase | CdGDH2 | Amino acid | SEQ ID NO.12 |
| Phenylpyruvate decarboxylase | ARO10 | Amino acid | SEQ ID NO.7 |
| α-keto acid decarboxylase | KDC | Amino acid | SEQ ID NO.8 |
| Aldehyde reductase | YahK | Amino acid | SEQ ID NO.9 |
| Aldehyde reductase | ADH | Amino acid | SEQ ID NO.10 |
| Phenylalanine transporter | PheP | Amino acid | SEQ ID NO.13 |
| Phenylalanine dehydrogenase | PheDH | Amino acid | SEQ ID NO.6 |
| Aromatic amino acid transaminase | TyrB | Nucleotide | SEQ ID NO.15 |
| Aromatic amino acid transaminase | ARO8 | Nucleotide | SEQ ID NO.90 |
| L-amino acid deaminase | LAAD | Nucleotide | SEQ ID NO.91 |
| Glutamate dehydrogenase | ScGDH2 | Nucleotide | SEQ ID NO.97 |
| Glutamate dehydrogenase | CdGDH2 | Nucleotide | SEQ ID NO.20 |
| Phenylpyruvate decarboxylase | ARO10 | Nucleotide | SEQ ID NO.18 |
| α-keto acid decarboxylase | KDC | Nucleotide | SEQ ID NO.95 |
| Aldehyde reductase | YahK | Nucleotide | SEQ ID NO.19 |
| Aldehyde reductase | ADH | Nucleotide | SEQ ID NO.96 |
| Phenylalanine transporter | PheP | Nucleotide | SEQ ID NO.21 |
| Phenylalanine dehydrogenase | PheDH | Nucleotide | SEQ ID NO.17 |
| Promoter | Pfnrs or PfnrS | Nucleotide | SEQ ID NO.22 |
| Promoter | Pfdhf | Nucleotide | SEQ ID NO.24 |
| Promoter | Pbba | Nucleotide | SEQ ID NO.98 |
| Promoter | Ptrc | Nucleotide | SEQ ID NO.99 |
| Promoter | Ptet | Nucleotide | SEQ ID NO.100 |
| Promoter | Pvan | Nucleotide | SEQ ID NO:101 |
| Promoter | PBAD | Nucleotide | SEQ ID NO:102 |
| Expression cassette | PvanRAM | Nucleotide | SEQ ID NO:103 |
| Expression cassette | Pfnrs-TyrB-gdh2 | Nucleotide | SEQ ID NO:104 |
| Expression cassette | Pfnrs-Aro10-yahk | Nucleotide | SEQ ID NO:105 |
| Expression cassette | Pfnrs-pheDH-pheP | Nucleotide | SEQ ID NO:106 |
| Expression cassette | Pvan-TyrB-gdh2 | Nucleotide | SEQ ID NO:107 |
| Expression cassette | Pvan-Aro10-yahk | Nucleotide | SEQ ID NO:108 |
| Expression cassette | Pvan-pheDH-pheP | Nucleotide | SEQ ID NO:109 |
| Expression cassette | PBAD-LAAD | Nucleotide | SEQ ID NO:110 |
| vanRAM repressor | vanRAM | Nucleotide | SEQ ID NO:111 |

**Table 2 sgRNA sequence information corresponding to the insertion site in gene editing**

| Insertion site | sgRNA sequence | No. |
|---|---|---|
| araAB | attgtggaaagaccacaccg | SEQ ID NO:112 |
| kefB | gccggaagacactatgaagc | SEQ ID NO:113 |
| maeB | acgcgcgcctcttccccttc | SEQ ID NO:114 |
| rhtB/C | tcatcagagtaagtcggata | SEQ ID NO:115 |
| agaI/rsml | tgtgtcgtcgttaactcgcc | SEQ ID NO:116 |
| ldhA | cgatccgtatccaagtgcgg | SEQ ID NO:117 |
| yjcs | cgtcgttaccccacatatcc | SEQ ID NO:118 |
| yghx | cgtatatcgaagatgtgacg | SEQ ID NO:119 |
| lacZ | cgaagccgccctgtaaacgg | SEQ ID NO:120 |
| kefB | gccggaagacactatgaagc | SEQ ID NO:121 |
| fliC | accatctttcagttgatcaa | SEQ ID NO:122 |
| bscA | ttcttccaccagtccacctt | SEQ ID NO:123 |
| nth/tppB | atattactggaacaacataa | SEQ ID NO:124 |
| thyA | gtttaccatacactggcccg | SEQ ID NO:125 |
| yics | ggtaaagaagcgttggtcag | SEQ ID NO:126 |
| malPT | cgctttttgaaaatacgcaa | SEQ ID NO:127 |
| yieN | gagggtgagccataatgaag | SEQ ID NO:128 |
| tkrA | ttcgaagacatccagcccgg | SEQ ID NO:129 |
| pflB | ccgtgacgttatccgcacca | SEQ ID NO:130 |

### Example 1: Editing scheme for bacterial gene

### 1.1 sgRNA design

A 20 bp sequence (N20NGG) linking the NGG PAM sequence was searched for on both strands of the target integration site sequence and blasted against the EcN genome. A unique 20 bp sequence was selected as the sgRNA for the target integration site. The 300-500 bp sequences upstream and downstream of the sgRNA were selected as the left homology arm (LHA) and right homology arm (RHA).

### 1.2 sgRNA plasmid construction

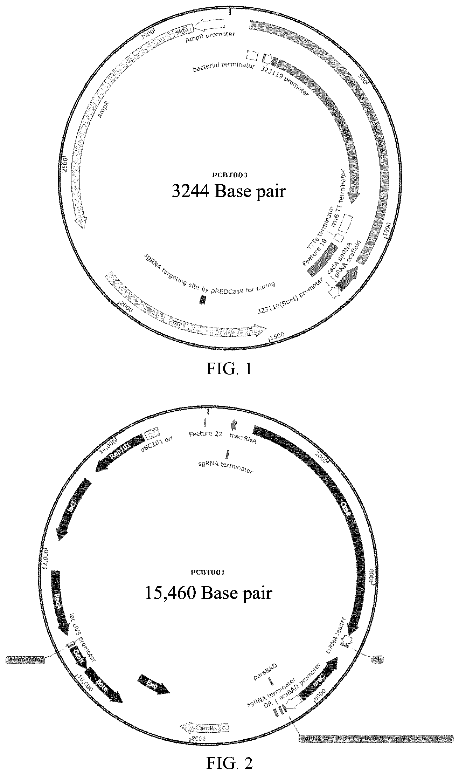

The sgRNA sequence was added to the 5' end of the reverse primer of the gRNA backbone, amplified by PCR and digested with restriction endonucleases PstI and SpeI. The digested PCR product fragment was ligated to the equally digested plasmid pCBT003 (SEQ ID NO: 84, the plasmid map as shown in Fig. 1) to form the pCBT003_sgRNA plasmid.

### 1.3 Preparation of donor gene fragments

The target gene to be integrated into the EcN genome was synthesized on a cloning plasmid (such as pUC57) by GeneScript. The target gene was amplified using the synthesized plasmid as a template, and the LHA and RHA of the selected integration site were amplified using the EcN genome as a template. The PCR primers used to amplify these fragments had 15-20 bp of homologous sequences with each other, so they could be linked by overlapping PCR, and the resulting PCR product containing LHA-target gene-RHA was used as the donor gene fragment.

### 1.4 Preparation of donor plasmid PCBT003-target gene-sgRNA

The pCBT003_sgRNA plasmid obtained from 1.2 expressing the sgRNA at the integration site, and the PCR product containing LHA-target gene-RHA obtained from 1.3 were ligated using the ClonExpress^{®} MultiS One-Step Cloning Kit, and the ligated product was transformed into Top10 competent cells, and the cells were screened with ampicillin-resistant plates (100 µg/mL); transformants were picked for colony PCR verification and sequencing, and the correctly sequenced PCBT003-target gene-sgRNA recombinant plasmids were selected.

### 1.5 Preparation of EcN/pCBT001 electrotransformed competent cells

The PCBT001 plasmid (a plasmid expressing Cas9 protein, SEQ ID NO.85, the plasmid map as shown in Fig. 2) was electrotransformed into E. coli EcN chemically competent cells, and the cells were screened on LB solid plates containing spectinomycin hydrochloride and streptomycin (50 µg/mL) to obtain the EcN/PCBT001 transformants.

A single colony of EcN/PCBT001 was picked into a LB liquid medium containing spectinomycin and streptomycin (50 µg/mL), and cultured overnight at 30°C and 220 rpm. On the next day, the bacterial liquid was added to 30 mL of LB liquid medium at an inoculum volume of 1 : 100 (V/V). When the bacterial concentration OD600 was 0.2, IPTG at a final concentration of 1 mM was added for induction, and the culture was continued for 2-3 h until the OD value was 0.6-0.8, and the electrotransformed competent cells were prepared.

### 1.6 Electrotransformation

The PCBT003-target gene-sgRNA plasmid was electrotransformed into EcN/PCBT001 competent cells (electrotransformation conditions: 2.5 kV, 200 S2, 25 µF), and the cells were spread on LB plates containing ampicillin (50 µg/mL), spectinomycin and streptomycin (50 µg/mL), and cultured for 1 d. Single colonies grown on the resistant plates were picked.

### 1.7 Elimination of PCBT003-target gene-sgRNA plasmid and pCBT001 plasmid

Single colonies that had successfully integrated the target gene expression cassette were picked and inoculated in LB liquid culture medium supplemented with spectinomycin and streptomycin (50 µg/mL), induction was performed by adding 10 mM of L-arabinose for 20-24 h at 30°C, and spectinomycin and streptomycin (50 µg/mL) LB plates were spread with the obtained bacterial liquid. The single colonies that grew were spotted on ampicillin, spectinomycin, and streptomycin (50 µg/mL) plates to observe the growth of the strains. The strains cannot grow on ampicillin-resistant plates, and the colonies that grow on spectinomycin and streptomycin plates were the strains with PCBT003-target gene-sgRNA eliminated.

The colonies with PCBT003-target gene-sgRNA plasmid eliminated were picked, inoculated into LB test tubes, and cultured at 42°C for 24 h, the obtained culture was diluted in gradients, spread on LB plates, and cultured overnight. The next day, single colonies were picked, streaked on spectinomycin and streptomycin-resistant plates, and the growth of the colonies was observed. The colonies that cannot grow on the spectinomycin and streptomycin plates are the strains with the PCBT001 plasmid eliminated.

### Example 2: Construction scheme of auxotrophic strains

The thyA gene was knocked out through CRISPR/Cas9 technology to achieve the deletion of the thyA gene in the EcN strain. Taking the construction of an EcN_ΔthyA strain as an example, the specific construction scheme was as follows: according to the construction scheme of Example 1, a recombinant plasmid PCBT003-thyA-HA-sgRNA comprising a donor gene fragment thyA-HA and the sgRNA targeting the thyA site (the sequence of thyA-HA-sgRNA as shown in SEQ ID NO: 131) was first constructed, and then it was used together with the pCBT001 plasmid expressing Cas9 protein to electrotransform the competent EcN strain, and after removing the excess plasmid, an auxotrophic EcN_ΔthyA strain was obtained.

Sequence of thyA-HA-sgRNA (SEQ ID NO: 131):

ATGGTGAACTGCCATGACAGGTATTCGCCCCAGCAACGGAACTCACAAGGCA
CCATAACGTCCCCTCCCTGATAAGACTGATACTGTGGTCGCGGTTATGCCAGTTGGCA
TCTTCACGTAAATAGAGCAAATAGTCCCGCGCCTGGCTGGCGGTTTGCCAAAGCCGT
TGCGACTGCTGCCAGTATTGCCAGCCATAGAGCCCACTTGCGCTTAGCATGACCAGAA
TCAGCATCGCGACCAGCGTTTCAATCAGCGTATAACCACGTTGTGTTTTCATGCCGGC
AGTATGGAGCGAGGAGAAAAAAAGACGAGGGCCAGTTTCTATTTCTTCGGCGCATCT
TCCGGACTATTTACGCCGTTGCAGGACGTTGCAAAATTTCGGGAAGGTGTCTCGAAG
AATTTAACGGAGGGCAAAAAAAACCGACGCACACTGGCGTCGGCTCTGGCAGGATG
TTTCGTAAGGTTCCTCAGGAAACGTGTTGCTGTGGGCTGCGACGATATGCCCAGACCA
TCATGATCACACCCGCGACAATCATCGGGATGGAAAGAATTTGCCCCATGCTGATGTA
CTGCACCCAGGCACCAGTAAACTGCGCGTCGGGCTGGCGGAAAAACTCAACAATGA
TGCGAAACGCGCCGTAACCAATCAGGAACAAGCCTGAGACAGCTCCCATTGGGCGC
GGTTTACGAATATACAGGTTGAGGATAATAAACAGCACCACACCTTCCAGCAGCAGCT
CGTAAAGCTGTGATGGGTGGCGCGGCAGCACACCGTAAGTGTCGAAAATGGATTGCC
ACTGCGGGTTGGTTTGCAGCAGCAAAATATCTTCTGTACGGGAGCCAGGGAACAGCA
TGGCAAACGGGAAGTTCGGGTCAACGCGGCCCCACAATTCACCGTTAATAAAGTTGC

CCAGGCGCCCAGCACCAAGACCAAACGGAATGAGTGGGGCctgcaggaattcaaaaaaagcaccg
actcggtgccactttttcaagttgataacggactagccttattttaacttgctatttctagctctaaaacCGGGCCAGTGTATGGTA
AACactagtattatacctaggactgagctagctgtcaa

The ununderlined part in capitals is the left homology arm, the underlined part in capitals is the right homology arm, and the boxed part in capitals is the sgRNA sequence.

### Example 3: Knockout scheme of flagellar gene

In order to prolong the residence time of the strain in the intestine, the CRISPR/Cas9 gene editing system was used to knock out the key gene fliC of the flagellar system and the adhesion-related gene (cellulose synthase gene) bscA. Taking the construction of EcN_ΔthyAΔbscAΔfliC as an example, the specific construction scheme was as follows: firstly, a competent EcN_ΔthyA strain was prepared, and then the recombinant plasmid PCBT003-fliC-HA-sgRNA/PCBT003-bscA-HA-sgRNA (the sequence of fliC-HA-sgRNA as set forth in SEQ ID NO: 132, and the sequence of bscA-HA-sgRNA as set forth in SEQ ID NO: 133) containing the donor fragment and sgRNA targeting fliC or bscA were constructed, respectively, and then they were used together with the pCBT001 plasmid expressing Cas9 protein to electrotransform the EcN strain, and after removing the excess plasmid, an auxotrophic **EcN_ΔthyAΔbscAΔfliC** strain was obtained.

Sequence of fliC-HA-sgRNA (SEQ ID NO: 132):

AAGTTCATCCAGCATAGCGCCACGGATACGCTGCACTGCGTAAGTTGTAAATG
CCGTTCCTTGTAGGGCGTCATAGCGTTCGACGGCATTAAGTAACCCAATGCCGCCCGC
CTGTAGCAGATCGTCAAGTTCCACGCTCGCGGGCAGTCGAACCTGCAGGCGCAATGC
TTCGTGACGCACCAGCGGGACATAACGCTGCCACAGCGAGTGTTTATCCATTACACCT
TCAGCGGTATAGAGTGAATTCACGATAAACAGCCCTGCGTTATATGAGTTATCGGCATG
ATTATCCGTTTCTGCAGGGTTTTTAATCGGACGATTAGTGGGTGAAATGAGGGGTTATT
TGGGGGTTACCGGTAAATTGCGGGCAGAAAAAACCCCGCCGTTGGCGGGGAAGCAC
GTTGCTGGCAAATTACCATTCATGTTGCCGGATGCGGCGTAAACGCCTTATCCGGCCT
ACAAAAATGTGCAAATTCAATAAATTGCAATTCCCCTTGTAGGCCTGATAAGCGCAGC
GCATCAGGCAATTTGGCGTTGCCGTCAGTCTCAGTTAATCAGGTTACGGCGAGATTCG
TTATCCTATATTGCAAGTCGTTGATTACGTTTTGGGTTTCCACCCGTCGGCTCAATCGC
CGTCAACCCTGTTATCGTCTGTCGTAAAACAACCTTTAGAATTTTTTTCACAAACAGC
CATTTTTTGTTAGTCGACGAAATACTCTTTTCTCTGCCCCTTATTCCCGCTATTAAAAA
AAACAATTAAACGTAAACTTTGCGCAATTCAGGCCGATAACCCCGGTATTCGTTTTAC
GTGTCGAAAGATAAAAGGAAATCGCATGGCAAGTATTTCATCGCTGGGAGTCGGGTC
AGGTCTGGATTTAAGTTCAATCCTTGATAGCCTCACCGCCGCGCAAAAAGCGACGCT

AACCCCTATTTCAAATCAGCAATCGTCGTTTACCGCTAAACTTAGCGCCTACGGTACG
CTGAAAAGCGCGCTGACGACTTTCCAGACCGCCAATACTGCATTGTCTAAAGCCGAT
CTTTTTTCCGCTACCAGCACCACCAGCAGCACCACCGCGTTCAGTGCCACCACCGCG
GGTAATGCCATCGCCGGGAAATACACCATCAGCGTCACCCATCTGGCGCAGGCGCAA
ACCCTGACAACGCGCACCACCAGAGACGATACGAAAACGGCGATCGCCACCAGCGA
CAGCAAACTCACCATTCAACAAGGCGGCTatatactagaagcggccgctgcaggaattcaaaaaaagcaccga
ctcggtgccactttttcaagttgataacggactagccttattttaacttgctatttctagctctaaaacTTGATCAACTGAAAGAT
GGTactagtattatacctaggactgagctagctgtcaa

The ununderlined part in capitals is the left homology arm, the underlined part in capitals is the right homology arm, and the boxed part in capitals is the sgRNA sequence.

Sequence of bscA-HA-sgRNA (SEQ ID NO: 133):

AGGTCATTCTTCACATTCAGGGTCTGATAGGTCAGATCCAGTCCACTGCTCCGC
CCAACATCCAGCCAAAGCGTGGTGCTGGCCGGGTTTTCGCACACGTCCTGATAATGG
CCGACAAACTCCAGCCGCACACGGTTGAAATCAGTAATAAACAGCGGGTTAATGGGC
ATTTGTGCCAGCGTTTTTTTACCCAACTGTTCTTTGGTCACTGGCAGCACGCCCATCA
GTTCATCATTGAGATAAACCTTTAACTGCGACTGGACAGGCAGTAATGATGGCGATGG
CGTGTATTCGAGGTTGAGCATCGCCTTCGTCACCACTTCATCGCTGCGCATACCAAAC
TCAATGCTGCCGTTCGGGTTAATGCCACGTAGCACCATGCTGCCCGGCGGCGGCGCA
ATTTGCGCAAAGGTCAGCTTCACATCACGGGAAGGACCGTTCTGCGCCACGACTGGC
GCATCTGCGCCCTGCACGCCCGGCATCACTTGCCCCACCTGCGGATTTTGCTCCGCCT
GGGCGGCTACAGCTGGCTCAGCATTGATCAGTGGTTGCGTTGCTGGCGTCGCCTGCG
TCATGAAAGAGGGGAACGCACTCATCCCCAGAGCCACTGCACAAATCCAGAATATTT
TTCTTTTCATCGCGTTATCATCATGATGCACTCCCGACTGGCGTTTTCAGCCCGGAATA
GTTTAACAGGCACCAGTTAGCCAGCGTCAGTATTTCTTCAGCCGCCAGCGCATCACTG
CGAAATTCACCGACTGGTTGCTTAGCTGCCAGACATTCAGCCATCGCTTCATCACGAT
GAATGAGCATCGGCAGCAATCGGCGCTGGCTTTGCAACCAAAGCTGGTAAATATCGT
CCTGAACCTGGCTGCCAATACGGAAGTCATTAATCAAAATATGTGCACCATCCGGCAG
CGCTTGCTGATGCAGTCGGATATGGCAGTTGGCATCCACATTGACGATTGCCAGCGAG
TGATCGCACAAACTCAGCAGCTGGTGGGTTATCTGCGAGGCATCACGCGGTAAGTCA
ATTAAAATCCACTGGTAACGCCCGCTGGCTTTTAGTTGCTGTAAGCCGGAGCAAATAT
CGCTCAGCCGGGTTTGCCAGTGCTGTGGATTTTCTTGTTCTTCAATGGATAACTGACC
AAAAGGCAGCAAATCGAGCTGCGAGGTATAGCGTAACCCGGCGTCACGCCAGTCCTG
GCCATCCAGCATAGCTCTGGCCCAACCCTGACGGTGGGTAAAATCAACGTTAAATGA
CAGGCGCAACAAGTTGTCCGGGCAGGtatatactagaagcggccgctgcaggaattcaaaaaaagcaccgactc

ggtgccactttttcaagttgataacggactagccttattttaacttgctatttctagctctaaaacAAGGTGGACTGGTGGAAG
AAactagtattatacctaggactgagctagctgtcaa

The ununderlined part in capitals is the left homology arm, the underlined part in capitals is the right homology arm, and the boxed part in capitals is the sgRNA sequence.

### Example 4: Construction of engineered strains that degrade phenylalanine

An engineered strain capable of degrading phenylalanine was obtained by knocking in relevant genes that are capable of converting phenylalanine to products without toxic side effects, using EcN as the chassis bacterium or a strain constructed in Example 2 or 3 that has been knocked out of the flagellar genes and/or was auxotrophic as the chassis bacterium.

The sequence of the expression cassette used in the construction process is as follows: vanRAM repressor (SEQ ID NO: 103), Pfnrs-TyrB-gdh2 (SEQ ID NO: 104), Pfnrs-Aro10-yahk (SEQ ID NO:105), Pfnrs-pheDH-pheP (SEQ ID NO: 106), Pvan-TyrB-gdh2 (SEQ ID NO: 107), Pvan-Aro10-yahk (SEQ ID NO: 108), Pvan-pheDH-pheP (SEQ ID NO: 109), PBAD-LAAD (SEQ ID NO: 110).

Optional insertion sites for the target gene expression cassette in EcN are selected from: one or more of yicS site, malPT site, maeB site, nth/tppB site, tkrA site, malE site, exo site, rhtB/C site, agaI/rsml site, araBD site, yghx site, ldhA site, araAB site, lacZ site and/or kefB site.

### 4.1 Construction of engineered strain CBT2001 that degrades phenylalanine

The genome of strain *E.coli* Nissle1917 (EcN) was subjected to engineering which includes the following steps:

### 4.1.1 Knock-in of Pfnrs-TyrB-gdh2 expression cassette at yghX site

### (1) Construction of PCBT003-yghX-sgRNAplasmid

Construction purpose: The sequence "catcgccgcagcggtttcag" of the original target site X in the PCBT003 plasmid was replaced with the new N20 sequence "cgtatatcgaagatgtgacg" (yghX) containing the target site X, so as to construct the PCBT003 plasmid (PCBT003-yghX-sgRNA) containing" cgtatatcgaagatgtgacg".

Construction method:
1) Design of primer yghX-sgRNA-f (PstI): SEQ ID NO.25
   5'-**ctgcag**gaattcaaaaaaagcaccgactcggtg-3' (the bold lowercase font is the PstI restriction site sequence, other sequences are the sequences on the PCBT003 plasmid)
2) Design of primer yghX-sgRNA-r (SpeI): SEQ ID NO.26
   5' -**actagtCGTATATCGAAGATGTGACG**gttttagagctagaaatagcaagtta-3' (the bold lowercase font is the SpeI restriction site sequence, the bold uppercase font is the N20 sequence of the yghx site, other sequences are the sequences on the PCBT003 plasmid)
3) Primers yghX-sgRNA-f (SEQ ID NO.25) and yghX-sgRNA-r (SEQ ID NO.26) were used to amplify N20 sequence fragment containing target site X (yghX) from the DNA template of PCBT003 plasmid (SEQ ID NO.84) by PCR, the resulting fragment was digested with restriction endonucleases PstI and speI, and the PCR product (121 bp) was purified and recovered. The PCBT003 plasmid was digested using PstI enzyme and speI enzyme, and the digested fragment (3123 bp) recovered from the gel was ligated to the purified and recovered PCR product to construct the PCBT003 plasmid (PCBT003-yghX-sgRNA) containing the target site X (yghX) sequence, which was then transformed into *E. coli* Top10 competent cells. An ampicillin-resistant plate (100 µg/mL) was used for screen, transformants were picked and cultured under shaking, the plasmids were extracted, and the primer PCBT003-seq-r (SEQ ID NO. 27) was used for sequencing. The correctly sequenced sgRNA plasmids were selected respectively: PCBT003-yghX-sgRNA.

(2) Preparation of plasmid PCBT003-Pfnrs-TyrB-gdh2-yghX-sgRNA for electrotransformation:
Restriction endonucleases PstI and XbaI were used to digest the PCBT003-yghX-sgRNA plasmid obtained above, and gel recovery was performed on the digested large fragment (plasmid backbone). The amplification primers yghX-LH-f (SEQ ID NO.30), yghX-LH-r1 (SEQ ID NO.31), yghX-RH-f1 (SEQ ID NO.32) and yghX-RH-r (SEQ ID NO.33) were used to amplify the upstream homology arm fragment and the downstream homology arm fragment of the yghx site from the genome template of strain E. coli Nissle1917, and the PCR products were purified. The pUC-fnrs-TyrB-gdh2 plasmid (SEQ ID NO.86) (synthesized by GenScript) was used as a template, and fnrs-TyrB-f1 (SEQ ID NO.28) and gdh2-r1 (SEQ ID NO.29) were used as primers for PCR amplification to obtain the Pfnrs-TyrB-gdh2 fragment of about 5260 bp. The digested fragments (plasmid backbone) recovered from the gel, the purified and recovered upstream and downstream homology arm PCR products and the Pfnrs-TyrB-gdh2 fragment were ligated using the ClonExpress^{®} MultiS One-Step Cloning Kit, the ligated product was transformed into Top10 competent cells, and the cells were screened with ampicillin-resistant plates (100 µg/mL); transformants were picked for colony PCR verification and sequencing, and the correctly sequenced PCBT003-Pfnrs-TyrB-gdh2-yghX-sgRNA recombinant plasmids were selected.

(3) Preparation method of competent cells for electrotransformation:
A single colony of EcN was picked into 5 mL of LB liquid medium, and cultured overnight at 37°C and 220 rpm. The next day, the bacterial liquid was added to 30 mL of LB liquid medium at an inoculation volume of 1 : 100, and culture was performed with shaking for 2-3 h until the OD value was 0.6-0.8; the bacterial liquid was poured into a pre-cooled centrifuge tube which was kept under ice bath for 15-20 min; then the centrifuge tube was centrifuged at 6000 rpm and 4°C for 7 min, the supernatant was discarded, and 20 mL of 10% pre-cooled glycerol was added to suspend the bacteria evenly; then centrifuging and discarding supernatant were repeat for three times; the supernatant was discarded, 300 µL of 10% pre-cooled glycerol was added to suspend the bacteria evenly, and the suspended bacterial liquid was dispensed into centrifuge tubes at 100 µL per tube.

The PCBT001 plasmid (SEQ ID NO.85) was electrotransformed into E. coli EcN chemically competent cells, and the cells were screened on LB solid plates containing spectinomycin hydrochloride (50 µg/mL) to obtain the EcN/PCBT001 transformants.

A single colony of EcN/PCBT001 was picked into a LB liquid medium containing spectinomycin (50 µg/mL), and cultured overnight at 30°C and 220 rpm. On the next day, the bacterial liquid was added to 30 mL of LB liquid medium at an inoculum volume of 1 : 100 (V/V). When the bacterial concentration OD600 was 0.2, IPTG at a final concentration of 1 mM was added for induction, and the culture was continued for 2-3 h until the OD value was 0.6-0.8, and the electrotransformed competent cells were prepared.

### (4) Electrotransformation:

The PCBT003-Pfnrs-TyrB-gdh2-yghX-sgRNA plasmid was electrotransformed into EcN/PCBT001 competent cells (electrotransformation conditions: 2.5 kV, 200 Ω, 25 µF), and the cells were spread on LB plates containing ampicillin (50 µg/mL) and spectinomycin (50 µg/mL), and cultured for 1 d. A single colony growing on the resistant plate was picked and the primers yghX-verify-f (SEQ ID NO.34) and yghX-verify-r (SEQ ID NO.35) were used to verify the integration of the gene through colony PCR, and the positive fragment was about 6662 bp;

### (5) Elimination of PCBT003-Pfnrs-TyrB-gdh2-yghX-sgRNA plasmid:

Single colonies that had successfully integrated the Pfnrs-TyrB-gdh2 expression cassette were picked and inoculated in LB liquid culture medium supplemented with spectinomycin (50 µg/mL), induction was performed by adding 10 mM of L-arabinose for 20-24 h, and spectinomycin LB plates were spread with the obtained bacterial liquid. The single colonies that grew were spotted on ampicillin and spectinomycin plates to observe the growth of the strains. The strains cannot grow on ampicillin-resistant plates, and the colonies that grew on spectinomycin-resistant plates were the strains with PCBT003-Pfnrs-TyrB-gdh2-yghX-sgRNA eliminated, and EcN_Δyghx::Pfnrs-TyrB-gdh2/PCBT001 was obtained.

### 4.1.2 Knock-in of Pfnrs-ARO10-YahK expression cassette at ldhA site

### (1) Construction of PCBT003-ldhA-sgRNAplasmid

primers ldhA-sgRNA-f (SEQ ID NO.36) and ldhA-sgRNA-r (SEQ ID NO.37) were used to amplify N20 sequence fragment containing target site X (ldhA) from the template of PCBT003 plasmid by PCR, the resulting fragment was digested with restriction endonucleases PstI and speI, and the PCR product was purified and recovered.

The PCBT003 plasmid was digested using PstI enzyme and speI enzyme, and the digested fragment (about 3123 bp) recovered from the gel was ligated to the purified and recovered PCR product and the ligated product was then transformed into E. coli Top10 competent cells. Screen was performed on ampicillin-resistant plate (100 µg/mL) to obtain PCBT003-ldhA-sgRNA plasmid.

(2) Preparation of plasmid PCBT003-Pfnrs-ARO10-YahK-ldhA-sgRNA for electrotransformation:
Restriction endonucleases PstI and XbaI were used to digest the PCBT003-ldhA-sgRNA plasmid, and gel recovery was performed on the digested large fragment (plasmid backbone).

The amplification primers ldhA-LH-f (SEQ ID NO.40) and ldhA-LH-r1 (SEQ ID NO.41) and ldhA-RH-f1 (SEQ ID NO.42) and ldhA-RH-r (SEQ ID NO.43) were used to amplify the upstream homology arm fragment and the downstream homology arm fragment of the ldhA site from the EcN genome template, and the amplified products were about 500 bp, respectively.

The pUC-fnrs-ARO10-YahK plasmid (SEQ ID NO.87) (synthesized by GenScript) was used as a template, and fnrs-ARO10-f1 (SEQ ID NO.38) and YahK-r1 (SEQ ID NO.39) were used as primers for PCR amplification to obtain the Pfnrs-ARO10-YahK fragment of about 4779 bp.

The digested fragment (plasmid backbone) recovered from the gel, the purified and recovered upstream and downstream homology arm PCR products and the Pfnrs-ARO10-YahK fragment were ligated using the ClonExpress^{®} MultiS One-Step Cloning Kit, the ligated product was transformed into Top10 competent cells, and the cells were screened with ampicillin-resistant plates (100 µg/mL); and the PCBT003-Pfnrs-TyrB-gdh2-ldhA-sgRNA recombinant plasmid was obtained.

(3) Referring to the method in 4.1.1(3), EcN_Δyghx::Pfnrs-TyrB-gdh2/PCBT001 competent cells for electrotransformation were prepared.

(4) Referring to the method in 4.1.1(4), the PCBT003-Pfnrs-ARO10-YahK-ldhA-sgRNA plasmid was electrotransformed into EcN_Δyghx::Pfnrs-TyrB-gdh2/PCBT001 competent cells, and transformants were verified by colony PCR using ldhA-verify-f (SEQ ID NO.44) and ldhA-verify-r (SEQ ID NO.45)

(5) Referring to method 4.1.1(5), the helper plasmid was eliminated to obtain EcN_Δyghx::Pfnrs-TyrB-gdh2_Δldha::Pfnrs-ARO10-YahK/PCBT001.

### (6) Elimination of PCBT001 plasmid

The colonies with PCBT003-Pfnrs-TyrB-gdh2-kefB-sgRNA plasmid eliminated were picked, inoculated into LB test tubes, and cultured at 42°C for 24 h, the obtained culture was diluted in gradients, spread on LB plates, and cultured overnight. The next day, single colonies were picked, streaked on spectinomycin-resistant plates, and the growth of the colonies was observed. The colony that cannot grow on the spectinomycin plate was the strain EcN_Δyghx::Pfnrs-TyrB-gdh2_Δldha::Pfnrs-ARO10-YahK with PCBT001 plasmid eliminated, named CBT2001.

Through the above examples, the engineered bacterium CBT2001 was finally obtained, which was a derivative of E. coli Nissle1917, and has integrated the single-copy aromatic amino acid transaminase gene TyrB, the glutamate dehydrogenase gene gdh2, the aldehyde reductase gene YahK, and the phenylpyruvate decarboxylase gene ARO10, respectively, into the genome.

Genotype of engineered bacterium CBT2001: Nissle_Δyghx::Pfnrs-TyrB-gdh2_ Δldha: :Pfnrs-ARO10-YahK.

### 4.2 Construction of engineered strain CBT2002 that degrades phenylalanine

### 4.2.1 Knock-in of Pfnrs-TyrB-gdh2 at araAB site

### (1) Construction of PCBT003-araAB-sgRNA plasmid

Primers araAB-sgRNA-f (SEQ ID NO.46) and araAB-sgRNA-r (SEQ ID NO.47) were used to amplify N20 sequence fragment containing target site X (araAB) from the template of PCBT003 plasmid by PCR, the resulting fragment was digested with restriction endonucleases PstI and speI, and the PCR product was purified and recovered.

The PCBT003 plasmid was digested using PstI enzyme and speI enzyme, and the digested fragment (about 3123 bp) recovered from the gel was ligated to the purified and recovered PCR product and the ligated product was then transformed into E. coli Top10 competent cells. Screen was performed on ampicillin-resistant plate (100 µg/mL) to obtain PCBT003-araAB-sgRNA plasmid.

(2) Preparation of plasmid PCBT003-Pfnrs-TyrB-gdh2-araAB-sgRNA for electrotransformation:
Restriction endonucleases PstI and XbaI were used to digest the PCBT003-araAB-sgRNA plasmid, and gel recovery was performed on the digested large fragment (plasmid backbone).

The amplification primers araAB-LH-f (SEQ ID NO.50) and araAB-LH-r (SEQ ID NO.51) and araAB-RH-f (SEQ ID NO.52) and araAB-RH-r (SEQ ID NO.53) were used to amplify the upstream homology arm fragment and the downstream homology arm fragment of the araAB site from the EcN genome template, and the amplified products were about 500 bp, respectively.

The pUC-fnrs-TyrB-gdh2 plasmid (SEQ ID NO.86) (synthesized by GenScript) was used as a template, and fnrs-TyrB-f2 (SEQ ID NO.48) and gdh2-r2 (SEQ ID NO.49) were used as primers for PCR amplification to obtain the Pfnrs-TyrB-gdh2 fragment of about 4779 bp. The digested fragment (plasmid backbone) recovered from the gel, the purified and recovered upstream and downstream homology arm PCR products and the Pfnrs-TyrB-gdh2 fragment were ligated using the ClonExpress^{®} MultiS One-Step Cloning Kit, the ligated product was transformed into Top10 competent cells, and the cells were screened with ampicillin-resistant plates (100 µg/mL); and the PCBT003-Pfnrs-TyrB-gdh2-araAB-sgRNA recombinant plasmid was obtained.

(3) Referring to the method in 4.1.1(3), EcN_Δyghx::Pfnrs-TyrB-gdh2_Δldha::Pfnrs -ARO10-YahK/PCBT001 competent cells for electrotransformation were prepared.

(4) Referring to the method in 4.1.1(4), the PCBT003-Pfnrs-TyrB-gdh2-araAB-sgRNA plasmid was electrotransformed into EcN_Δyghx::Pfnrs-TyrB-gdh2_Δldha::Pfnrs -ARO10-YahK/PCBT001 competent cells, and transformants were verified by colony PCR using araAB-verify-f (SEQ ID NO.54) and araAB-verify-r (SEQ ID NO.55)

(5) Referring to method 4.1.1(5), the helper plasmid was eliminated to obtain EcN_Δyghx::Pfnrs-TyrB-gdh2_Δldha::Pfnrs-ARO10-YahK_ΔaraAB::Pfnrs-TyrB-gdh2.

### 4.2.2 Knock-in of Pfnrs-ARO10-YahK at lacZ site

(1) Construction of PCBT003-lacZ-sgRNAplasmid
   primers lacZ-sgRNA-f (SEQ ID NO.56) and lacZ-sgRNA-r (SEQ ID NO.57) were used to amplify N20 sequence fragment containing target site X (lacZ) from the template of PCBT003 plasmid by PCR, the resulting fragment was digested with restriction endonucleases PstI and speI, and the PCR product was purified and recovered. The PCBT003 plasmid was digested using PstI enzyme and speI enzyme, and the digested fragment (about 3123 bp) recovered from the gel was ligated to the purified and recovered PCR product and the ligated product was then transformed into E. coli Top10 competent cells. Screen was performed on ampicillin-resistant plate (100 µg/mL) to obtain PCBT003-lacZ-sgRNA plasmid.
(2) Preparation of plasmid PCBT003-Pfnrs-ARO10-YahK-lacZ-sgRNA for electrotransformation:
   Restriction endonucleases PstI and XbaI were used to digest the PCBT003-lacZ-sgRNA plasmid, and gel recovery was performed on the digested large fragment (plasmid backbone).

The amplification primers lacZ-LH-f (SEQ ID NO.60) and lacZ-LH-r (SEQ ID NO.61) and lacZ-RH-f (SEQ ID NO.62) and lacZ-RH-r (SEQ ID NO.63) were used to amplify the upstream homology arm fragment and the downstream homology arm fragment of the lacZ site from the EcN genome template, and the amplified products were about 500 bp, respectively.

The pUC-fnrs-ARO10-YahK plasmid (SEQ ID NO.87) (synthesized by GenScript) was used as a template, and fnrs-ARO10-f2 (SEQ ID NO.58) and YahK-r2 (SEQ ID NO.59) were used as primers for PCR amplification to obtain the Pfnrs-ARO10-YahK fragment of about 4819 bp. The digested fragment (plasmid backbone) recovered from the gel, the purified and recovered upstream and downstream homology arm PCR products and the Pfnrs-ARO10-YahK fragment were ligated using the ClonExpress^{®} MultiS One-Step Cloning Kit, the ligated product was transformed into Top10 competent cells, and the cells were screened with ampicillin-resistant plates (100 µg/mL); and the PCBT003-Pfnrs-ARO10-YahK-lacZ-sgRNA recombinant plasmid was obtained.

(3) Referring to the method in 4.1.1(3), EcN_Δyghx::Pfnrs-TyrB-gdh2_Δldha::Pfnrs-ARO10-YahK_ΔaraAB::Pfnrs-TyrB-gdh2/PCBT001 competent cells for electrotransformation were prepared.

(4) Referring to the method in 4.1.1(4), the PCBT003-Pfnrs-TyrB-gdh2-araAB-sgRNA plasmid was electrotransformed into EcN_Δyghx::Pfnrs-TyrB-gdh2_Δldha::Pfnrs-ARO10 -YahK_ΔaraAB::Pfnrs-TyrB-gdh2 competent cells, and transformants were verified by colony PCR using lacZ-verify-f (SEQ ID NO.64) and lacZ-verify-r (SEQ ID NO.65).

(5) Referring to method 4.1.1(5), the helper plasmid was eliminated to obtain EcN_Δyghx::Pfnrs-TyrB-gdh2_Δldha::Pfnrs-ARO10-YahK_ΔaraAB::Pfnrs-TyrB-gdh2_ΔlacZ:: Pfnrs-ARO10-YahK/PCBT001.

(6) Referring to method 4.1.2(6), the helper plasmid PCBT001 was eliminated to obtain EcN_Δyghx::Pfnrs-TyrB-gdh2_Δldha::Pfnrs-ARO10-YahK_ΔaraAB::Pfnrs-TyrB-gdh2_ΔlacZ:: Pfnrs-ARO10-YahK, named CBT2002.

Through the above examples, the engineered bacterium CBT2002 was finally obtained, which was a derivative of CBT2001 obtained by further integrating one copy of the aromatic amino acid transaminase gene, the glutamate dehydrogenase gene, the aldehyde reductase gene and the phenylpyruvate decarboxylase gene, respectively, into the CBT2001 genome.

Genotype of engineered bacterium CBT2002:
EcN_Δyghx::Pfnrs-TyrB-gdh2_Δldha::Pfnrs-ARO10-YahK_ΔaraAB::Pfnrs-TyrB-gdh2_ ΔlacZ::Pfnrs-ARO10-YahK

### 4.3 Construction of engineered strain CBT2003 that degrades phenylalanine

### 4.3.1 knock-in of Pfnrs-PheP at kefB site

### (1) Construction of PCBT003-kefB-sgRNAplasmid

Primers kefB-sgRNA-f (SEQ ID NO.66) and kefB-sgRNA-r (SEQ ID NO.67) were used to amplify N20 sequence fragment containing target site X (kefB) from the template of PCBT003 plasmid by PCR, the resulting fragment was digested with restriction endonucleases PstI and speI, and the PCR product was purified and recovered.

The PCBT003 plasmid was digested using PstI enzyme and speI enzyme, and the digested fragment (about 3123 bp) recovered from the gel was ligated to the purified and recovered PCR product and the ligated product was then transformed into E. coli Top10 competent cells. Screen was performed on ampicillin-resistant plate (100 µg/mL) to obtain PCBT003-kefB-sgRNA plasmid.

### (2) Preparation of plasmid PCBT003-Pfnrs-PheP-kefB-sgRNA for electrotransformation:

Restriction endonucleases PstI and XbaI were used to digest the PCBT003-kefB-sgRNA plasmid, and gel recovery was performed on the digested large fragment (plasmid backbone). The amplification primers kefB-LH-f (SEQ ID NO.70) and kefB-LH-r (SEQ ID NO.71) and kefB-RH-f (SEQ ID NO.72) and kefB-RH-r (SEQ ID NO.73) were used to amplify the upstream homology arm fragment and the downstream homology arm fragment of the kefB site from the EcN genome template, and the amplified products were about 500 bp, respectively.

The pUC-fnrs-PheP plasmid (synthesized by GenScript) was used as a template, and fnrs-PheP-f (SEQ ID NO.68) and PheP-r (SEQ ID NO.78) were used as primers for PCR amplification to obtain the Pfnrs-PheP fragment of about 1406 bp. The digested fragment (plasmid backbone) recovered from the gel, the purified and recovered upstream and downstream homology arm PCR products and the Pfnrs-PheP fragment were ligated using the ClonExpress^{®} MultiS One-Step Cloning Kit, the ligated product was transformed into Top10 competent cells, and the cells were screened with ampicillin-resistant plates (100 µg/mL); and the PCBT003-Pfnrs-PheP-kefB-sgRNA recombinant plasmid was obtained.

(3) Referring to the method in 4.1.1(3), EcN_Δyghx::Pfnrs-TyrB-gdh2_Δldha::Pfnrs-ARO10-YahK_ΔaraAB::Pfnrs-TyrB-gdh2_ΔlacZ:: Pfnrs-ARO10-YahK/PCBT001 competent cells for electrotransformation were prepared.

(4) Referring to the method in 4.1.1(4), the PCBT003-Pfnrs-PheP-kefB-sgRNA plasmid was electrotransformed into EcN_Δyghx::Pfnrs-TyrB-gdh2_Δldha::Pfnrs-ARO10-YahK_ΔaraAB::Pfnrs-TyrB-gdh2_ΔlacZ:: Pfnrs-ARO10-YahK/PCBT001 competent cells, and transformants were verified by colony PCR using kefB-verify-f (SEQ ID NO.74) and kefB-verify-r (SEQ ID NO.75).

(5) Referring to method 4.1.1(5), the helper plasmid PCBT003-Pfnrs-PheP-kefB-sgRNA was eliminated to obtain EcN_Δyghx::Pfnrs-TyrB-gdh2_Δldha::Pfnrs-ARO10-YahK_ ΔaraAB::Pfnrs-TyrB-gdh2_ΔlacZ::Pfnrs-ARO10-YahK_ΔkefB::Pfnrs-PheP/PCBT001.

(6) Referring to method 4.1.2(6), the helper plasmid PCBT001 was eliminated to obtain EcN_Δyghx::Pfnrs-TyrB-gdh2-TPheP_Δldha::Pfnrs-ARO10-YahK_ΔaraAB::Pfnrs-TyrB-gdh2_ ΔlacZ::Pfnrs-ARO10-YahK_ΔkefB::Pfnrs-PheP, named CBT2003.

The engineered strain CBT2003 finally obtained through the above examples was a derivative of CBT2002 and has single copy of the phenylalanine transporter gene further integrated into the genome of CBT2002 to enhance the uptake of phenylalanine by the strain.

Genotype of engineered bacterium CBT2003:
EcN_Δyghx::Pfnrs-TyrB-gdh2_Δldha::Pfnrs-ARO10-YahK_ΔaraAB::Pfnrs-TyrB-gdh2-_ ΔlacZ: :Pfnrs-ARO10-YahK_ΔkefB::Pfnrs-PheP

### 4.4 Construction of CBT-201 strain and derivative strains (the promoter is Pfnrs)

This engineered strain had the following introduced at different selected sites in the genome of a chassis bacterium: 2 copies of phenylalanine aminotransferase TyrB, single copy of phenylalanine dehydrogenase pheDH (from Bacillus SLBN-3, GenBank: TQJ42029.1) and single copy of L-amino acid deaminase (LAAD), so as to convert phenylalanine to phenylpyruvate; two copies of phenylpyruvate decarboxylase Aro10 so as to convert the phenylpyruvate generated in the previous step to phenylacetaldehyde; aldehyde reductase Yahk so as to further convert phenylacetaldehyde into phenylethanol; glutamate dehydrogenase GDH used to cooperate with TyrB to act in amino metabolism; and phenylalanine transporter pheP used to transfer phenylalanine outside the bacteria into the engineered bacteria. During the specific construction process, some genes are connected in series and use the same promoter to regulate transcription, thereby achieving control of gene expression quantity. The promoter used in the expression cassette was PfnrS. The specific construction process and strain structure are as follows:

### 4.4.1 Knock-in of Pfnrs-TyrB-gdh2 expression cassette at yghX and araAB sites

The Pfnrs-TyrB-gdh2 expression cassette (SEQ ID NO: 104) was synthesized by GeneScript. The sequences of sgRNAs targeting yghx and araAB are shown in Table 2. According to the methods of Examples 1 and 4, the sgRNA sequence and expression cassette were sequentially ligated into the pCBT003 vector respectively, thereby obtaining recombinant plasmids PCBT003-Pfnrs-TyrB-gdh2-yghX-sgRNA and PCBT003-Pfnrs-TyrB-gdh2 -araAB-sgRNA; subsequently, the above recombinant plasmids were electrotransformed together with the pCBT001 plasmid expressing Cas9 protein into the chassis bacterium EcNΔthyA respectively, and the excess plasmids were removed to obtain the recombinant bacterium EcNΔthyAΔyghX::Pfnrs-TyrB-gdh2ΔaraAB::Pfnrs-TyrB-gdh2.

### 4.4.2 Knock-in of Pfnrs-Aro10-yahk at ldhA and lacZ sites

The Pfnrs-Aro10-yahk expression cassette (SEQ ID NO: 105) was synthesized by GeneScript. The sequences of sgRNAs targeting ldhA and lacZ are shown in Table 2. According to the methods of Examples 1 and 4, the sgRNA sequence and expression cassette Pfnrs-Aro10-yahk were sequentially ligated into the pCBT003 vector respectively, thereby obtaining recombinant plasmids PCBT003-Pfnrs-Aro10-yahk-ldhA-sgRNA and PCBT003-Pfnrs-Aro10-yahk-lacZ-sgRNA; subsequently, the above recombinant plasmids were electrotransformed together with the pCBT001 plasmid expressing Cas9 protein into the engineered bacteria obtained in the previous step respectively, and the excess plasmids were removed to obtain the recombinant bacterium EcNΔthyAΔyghX::Pfnrs-TyrB-gdh2ΔldhA:: Pfnrs-ARO10-yahkΔaraAB: :Pfnrs-TyrB-gdh2ΔlacZ : :Pfnrs-ARO10-yahk.

### 4.4.3 Knock-in of PfnrS_pheDH_pheP at kefB site

The PfnrS_pheDH_pheP expression cassette (SEQ ID NO: 106) was synthesized by GeneScript. The codon-optimized pheDH gene sequence is as shown in SEQ ID NO: 17. The sgRNA sequence targeting kefB is as shown in Table 2. According to the methods of Examples 1 and 4, the sgRNA sequence and expression cassette were sequentially ligated into the pCBT003 vector, thereby obtaining recombinant plasmid PCBT003-PfnrS_pheDH_pheP-kefB-sgRNA; subsequently, the above recombinant plasmids were electrotransformed together with the pCBT001 plasmid expressing Cas9 protein into the engineered bacteria obtained in the previous step, and the excess plasmids were removed to obtain the recombinant bacterium EcNΔthyAΔyghX: :Pfnrs-TyrB-gdh2ΔldhA::Pfnrs-ARO10-yahkΔaraAB::Pfnrs-TyrB-gdh2ΔlacZ ::Pfnrs-ARO10-yahkΔkefB::Pfnrs-pheDH-phep.

### 4.4.4 Knock-in of PBAD_LAAD at rhtB/C site

The PBAD_LAAD expression cassette (SEQ ID NO: 110) was synthesized by GeneScript. The sequence of sgRNA targeting rhtB/C is shown in Table 2. According to the methods of Examples 1 and 4, the sgRNA sequence and expression cassette were sequentially ligated into the pCBT003 vector, thereby obtaining recombinant plasmid PCBT003-PBAD_LAAD-rhtB/C-sgRNA; subsequently, the above recombinant plasmids were electrotransformed together with the pCBT001 plasmid expressing Cas9 protein into the engineered bacteria obtained in the previous step, and the excess plasmids were removed to obtain the recombinant bacterium EcNΔthyAΔyghX::Pfnrs-TyrB-gdh2ΔldhA::Pfnrs-ARO10-yahkΔaraAB::Pfnrs-TyrB-gdh2ΔlacZ: :Pfnrs-ARO10-yahkΔkefB::Pfnrs-pheDH-phepΔr htB/C::PBAD_LAAD, i.e., the CBT-201 strain.

### Construction of CBT-201-AA

On the basis of the CBT-201 strain, two copies of the Pfnrs-ARO10-yahk expression cassette were further added to obtain the strain CBT-201-AA with a genotype as follows:
EcNΔthyAΔyghX::Pfnrs-TyrB-gdh2ΔldhA::Pfnrs-ARO10-yahkΔaraAB::Pfnrs-TyrB-gdh 2ΔlacZ::Pfnrs-ARO10-yahkΔkefB::Pfnrs-pheDH-phepΔagaI/rsml::Pfnrs-ARO10-yahkΔyjcS::Pf nrs-ARO10-yahkΔrhtB/C: :PBAD LAAD

### Construction of CBT-203 strain

According to the construction method for the CBT-201 strain, the same enzyme with the same copy number as that of the CBT-201 strain was transformed into the genome of the chassis bacterium EcNΔthyAΔbscAΔfliC to obtain the CBT-203 strain with a genotype as follows:
EcNΔthyAΔbscAΔfliCΔyghX::Pfnrs-TyrB-gdh2ΔldhA::Pfnrs-ARO10-yahkΔaraAB::Pfn rs-TyrB-gdh2ΔlacZ::Pfnrs-ARO10-yahkΔkefB::Pfnrs-pheDH-phepΔlacZ::Pfnrs-ARO10-yahkΔl acZ::Pfnrs-ARO10-yahkΔrhtB/C::PBAD LAAD

### 4.5 Construction of CBT-202, CBT-209, and CBT-210 strains:

Referring to the construction process for the CBT-201 strain, engineered strains CBT-202, CBT-209, and CBT-210 were constructed. The genotypes of the above engineered strains are as follows:
genotype of CBT-202: EcNΔthyAΔldhA::Pfnrs-pheDH-phepΔyghX::Pfnrs-ARO10-yahkΔmaeB::Pfnrs-pheDH-phepΔlacZ::Pfnrs-ARO10-yahkΔrhtB/C::PBAD_LAAD
genotype of CBT-209: EcNΔthyAΔldhA::Pfnrs-pheDH-phepΔyghX::Pfnrs-ARO10-yahk
genotype of CBT-210: EcNΔthyAΔyghX::Pfnrs-TyrB-gdh2ΔldhA::Pfnrs-ARO10-yahk△kefB::Pfnrs-pheP

### 4.6 Construction of CBT-206 strain (the promoter is Pvan):

The promoter used in all expression cassettes of CBT-206 strain is Pvan. The specific construction process and strain structure are as follows:

### 4.6.1 Knock-in of Pvan-TyrB-gdh2 expression cassette at yghX and araAB sites

The Pvan-TyrB-gdh2 expression cassette (SEQ ID NO: 107) was synthesized by GeneScript. The sequences of sgRNAs targeting yghx and araAB are shown in Table 2. According to the methods of Examples 1 and 4, the sgRNA sequence and expression cassette (Pvan-TyrB-gdh2 expression cassette, and vanRAM expression cassette as shown in SEQ ID NO: 103) were sequentially ligated into the pCBT003 vector respectively, thereby obtaining recombinant plasmids PCBT003-Pvan-TyrB-gdh2-yghX-sgRNA and PCBT003-Pvan-TyrB-gdh2-araAB-sgRNA; subsequently, the above recombinant plasmids were electrotransformed together with the pCBT001 plasmid expressing Cas9 protein into the chassis bacterium EcNΔthyAΔbscAΔfliC respectively, and the excess plasmids were removed to obtain the recombinant bacterium EcNΔthyAΔbscAΔfliCΔyghX::Pvan-TyrB-gdh2ΔaraAB:: Pvan-TyrB-gdh2.

### 4.6.2 Knock-in of Pvan-Aro10-yahk at ldhA, agaI/rsml and yjcS sites

The Pfnrs-Aro10-yahk expression cassette (SEQ ID NO: 108) was synthesized by GeneScript. The sequences of sgRNAs targeting ldhA, agaI/rsml and yjcS are shown in Table 2. According to the methods of Examples 1 and 4, the sgRNA sequence and expression cassette (Pvan-Aro10-yahk expression cassette, and vanRAM expression cassette as shown in SEQ ID NO: 103) were sequentially ligated into the pCBT003 vector respectively, thereby obtaining recombinant plasmids PCBT003- Pvan -Aro10-yahk-ldhA-sgRNA, PCBT003- Pvan -Aro10-yahk-agaI/rsml-sgRNA and PCBT003- Pvan -Aro10-yahk-yjcS-sgRNA; subsequently, the above recombinant plasmids were electrotransformed together with the pCBT001 plasmid expressing Cas9 protein into the engineered bacteria obtained in the previous step respectively, and the excess plasmids were removed to obtain the recombinant bacterium EcNΔthyAΔbscAΔfliCΔyghX::Pvan-TyrB-gdh2ΔyjcS::Pvan-ARO10-yahkΔaraAB::Pvan-TyrB-gdh2ΔldhA::Pvan-ARO10-yahkΔagaI/rsml::Pvan-ARO10-yahk.

### 4.6.3 Knock-in of Pvan _pheDH_pheP at nth/tppB site

Pvan_pheDH_pheP expression cassette (SEQ ID NO: 109) was synthesized by GeneScript. The sequence of sgRNA targeting nth/tppB is shown in Table 2. According to the methods of Examples 1 and 4, the sgRNA sequence and expression cassette (Pvan _pheDH_pheP expression cassette, and vanRAM expression cassette as shown in SEQ ID NO: 103) were sequentially ligated into the pCBT003 vector respectively, thereby obtaining recombinant plasmid PCBT003- Pvan_pheDH_pheP-nth/tppB-sgRNA; subsequently, the above recombinant plasmids were electrotransformed together with the pCBT001 plasmid expressing Cas9 protein into the engineered bacteria obtained in the previous step, and the excess plasmids were removed to obtain the recombinant bacterium EcNΔthyAΔbscAΔfliCΔyghX::Pvan-TyrB-gdh2ΔyjcS:: Pvan-ARO10-yahkΔaraAB::Pvan-TyrB-gdh2ΔldhA::Pvan-ARO10-yahkΔnth/tppB::Pvan-pheD H-phepΔagaI/rsml::Pvan-ARO10-yahk.

### 4.6.4 Knock-in of PBAD_LAAD at rhtB/C site

The PBAD_LAAD expression cassette (SEQ ID NO: 110) was synthesized by GeneScript. The sequence of sgRNA targeting rhtB/C is shown in Table 2. According to the method of Example 1, the sgRNA sequence and expression cassette (PBAD_LAAD expression cassette and vanRAM expression cassette as shown in SEQ ID NO: 103) were sequentially ligated into the pCBT003 vector, thereby obtaining recombinant plasmid PCBT003-PBAD_LAAD-rhtB/C-sgRNA; subsequently, the above recombinant plasmids were electrotransformed together with the pCBT001 plasmid expressing Cas9 protein into the engineered bacteria obtained in the previous step, and the excess plasmids were removed to obtain the recombinant bacterium EcNΔthyAΔbscAΔfliCΔyghX::Pvan-TyrB-gdh2ΔyjcS::Pvan-ARO 10-yahkΔaraAB: :Pvan-TyrB-gdh2ΔldhA: :Pvan-ARO10-yahkΔnth/tppB::Pvan-pheDH-phe pΔagaI/rsml::Pvan-ARO10-yahkΔrhtB/C::PBAD_LAAD, i.e., the CBT-206 strain.

### 4.7 Construction of CBT-205, CBT-207, and CBT-208 strains:

The construction was referred to the construction process for the CBT-206 strain, and the genotypes of CBT-205, CBT-207, and CBT-208 strains are as follows:
genotype of CBT-205 strain: EcNΔthyAΔyghX::Pvan-TyrB-gdh2ΔyjcS::Pvan-ARO10-yahkΔaraAB: :Pvan-TyrB-gdh2ΔldhA: :Pvan-ARO10-yahkΔnth/tppB::Pvan-pheDH-phepΔagaI/rs ml::Pvan-ARO10-yahkΔrhtB/C::PBAD_LAAD
genotype of CBT-207 strain: EcNΔthyAΔyghX::Pvan-TyrB-gdh2ΔyjcS::Pvan-ARO10-yahkΔaraAB::Pvan-TyrB-gdh2ΔldhA::Pvan-ARO10-yahkΔnth/tppB::Pvan-pheDH-phepΔagaI/rs ml::Pvan-ARO10-yahkΔtkrA::Pvan-pheDH-phepΔrhtB/C::PBAD_LAAD
genotype of CBT-208 strain: EcNΔthyAΔbscAΔfliCΔyghX::Pvan-TyrB-gdh2ΔyjcS:: Pvan-ARO10-yahkΔaraAB::Pvan-TyrB-gdh2ΔldhA::Pvan-ARO10-yahkΔnth/tppB::Pvan-pheD H-phepΔagaI/rsml::Pvan-ARO10-yahkΔtkrA::Pvan-pheDH-phepΔrhtB/C::PBAD_LAAD

### 4.8 Construction of CBT-212 and CBT-213 strains (the promoters are Pvan and Pfnrs)

Referring to the construction methods of Examples 4.4 and 4.6, strains CBT-212 and CBT-213 whose promoters are Pvan and Pfnrs were constructed, and their genotype information is as follows:
genotype of CBT-212 strain: EcNΔthyAΔyghX::Pvan-TyrB-gdh2ΔyjcS::Pvan-ARO10-yahkΔaraAB::Pfnrs-TyrB-gdh2ΔlacZ::Pfnrs-ARO10-yahkΔmaeB::Pfnrs-pheDH-phepΔagaI/rsml ::Pfnrs-ARO10-yahkΔrhtB/C::PBAD LAAD
genotype of CBT-213 strain: EcNΔthyAΔyghX::Pvan-TyrB-gdh2ΔyjcS::Pvan-ARO10-yahkΔaraAB::Pvan-TyrB-gdh2ΔldhA::Pvan-ARO10-yahkΔmaeB::Pfnrs-pheDH-phepΔagaI/rsml ::Pfnrs-ARO10-yahkΔkefB::Pfnrs-pheDH-phepΔrhtB/C::PBAD_LAAD

### Example 5: Detection method for the ability of engineered bacteria to degrade phenylalanine in vitro

### 5.1 HPLC detection method for phenylalanine and phenylethanol

The chromatographic column was Athena C18 liquid chromatographic column (250 mm × 4.6 mm, 5 µm); the column temperature was 35°C; 0.1% phosphoric acid in water (V/V) and methanol were used as the mobile phase to perform gradient elution; a UV detector was used with a detection wavelength of 254 nm and an injection volume of 10 µL.

### 5.2 In vitro activity detection method

The glycerinum bacterium EcN and each engineered bacterium were respectively inoculated into 5 mL of LB medium and cultured at 37°C overnight. The next day, the bacterial liquids were inoculated into 200 mL of LB medium with an inoculum volume of 1% (v/v) respectively, and cultured at 37°C and 220 rpm for 5.5 h. The OD₆₀₀ was determined, and the bacterial liquid was centrifuged at 4000 g for 8 min, the cells were collected, and resuspended in PBS buffer (containing 4 mM phenylalanine), and the bacterial concentration was adjusted to OD₆₀₀ of 2 or 0.5; 10 mL of the bacterial suspension was taken and placed in a 14 mL shaking tube respectively, marked as T = 0 h, and the shaking tube was placed at 37°C for culture at 220 rpm for 3 h, and then 1 mL of sample of was taken. The sample was centrifuged at 12,000 rpm for 10 min, and the supernatant sample was collected for HPLC detection.

### Example 6 Engineered bacteria with the phenylalanine degradation pathway therein can effectively degrade phenylalanine

This example used the method in Example 5 to test the ability of CBT2001/CBT2002 and CBT2003 to degrade phenylalanine in vitro. CBT2001 was an engineered bacterium that had integrated single copy of the phenylalanine metabolism pathway genes of the present disclosure into the genome of the probiotic E. coli Nissle1917 (including single copy of the aromatic amino acid transaminase gene TyrB, glutamate dehydrogenase gene gdh2, aldehyde reductase gene YahK, and phenylpyruvate decarboxylase gene ARO10), CBT2002 had integrated double copies of the phenylalanine metabolism pathway genes in CBT2001, and CBT2003 had further integrated a single-copy phenylalanine transporter gene in CBT2002 in order to enhance the strain's uptake of phenylalanine from the environment.

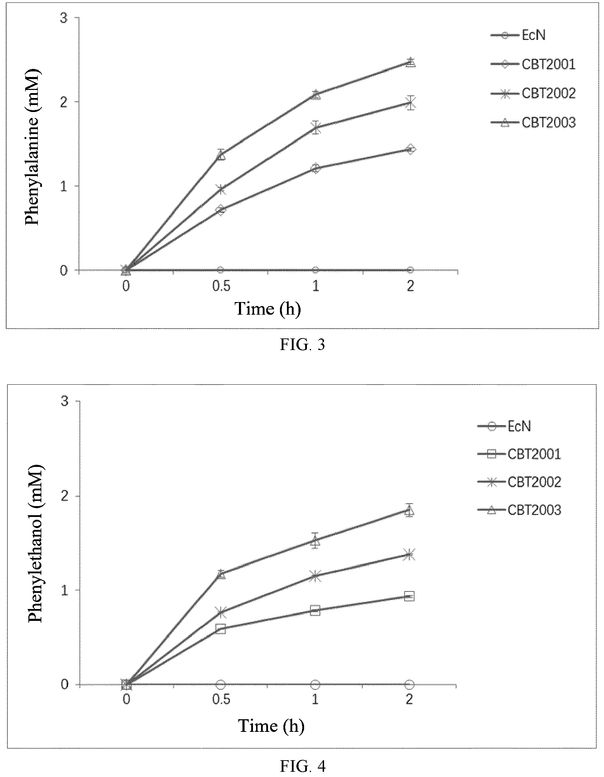

Under aerobic catalytic conditions, engineered strains CBT2001, CBT2002 and CBT2003 can all degrade phenylalanine to produce phenylethanol. The in vitro test results of phenylalanine degradation by each strain are shown in Table 3 and Fig. 3.

**Table 3 Maximum production rate of phenylethanol from phenylalanine metabolism by strains**

| Strains | Maximum production rate of phenylethanol (µmol/h/10⁹ cells) |
|---|---|
| CBT2001 | 3.27 ± 0.07 |
| CBT2002 | 4.41 ± 0.15 |
| CBT2003 | 6.29 ± 0.09 |

Under anaerobic catalytic conditions, the engineered strains degraded phenylalanine to produce phenylethanol. The in vitro test results of phenylalanine degradation by each strain are shown in Table 4 and Fig. 4.

**Table 4 Maximum production rate of phenylethanol from phenylalanine metabolism by strains**

| Strains | Maximum production rate of phenylethanol (µmol/h/10⁹ cells) |
|---|---|
| CBT2001 | 1.88 ± 0.06 |
| CBT2002 | 2.43 ± 0.04 |
| CBT2003 | 3.76 ± 0.07 |

As can be seen from Figs. 3 and 4, the original strain EcN was unable to degrade phenylalanine to produce phenylethanol under both aerobic and anaerobic catalytic conditions; whereas the engineered strains CBT2001, CBT2002 and CBT2003 all produced phenylethanol, indicating that the engineered bacteria of the present disclosure are capable of degrading phenylalanine and generating phenylethanol according to the design of the metabolic pathway of the engineered bacteria. Moreover, from the comparison of the metabolic capabilities of each strain, CBT2002, which had integrated two copies of related metabolic pathway genes, had stronger metabolic capabilities than CBT2001, which had integrated single copy. In addition, the incorporation of a phenylalanine transporter that can promote phenylalanine uptake made CBT2003 have stronger phenylalanine metabolism capabilities.

### Example 7 Optimization of engineered bacteria for phenylalanine metabolism

### 7.1 Phenylalanine dehydrogenase has better metabolic activity

In this example, an engineered strain CBT-209 was constructed that uses phenylalanine dehydrogenase to replace the aromatic amino acid transaminase gene TyrB, and the phenylalanine dehydrogenase was the only metabolizing enzyme that directly converted phenylalanine. Since transamination by TyrB no longer occurs, there is no need to add glutamate dehydrogenase for coupling the transamination reaction of phenylalanine with the reduction reaction of phenylacetaldehyde. Specifically, the CBT-209 strain contains single copies of phenylalanine dehydrogenase (pheDH), phenylpyruvate decarboxylase (Aro10), aldehyde reductase yahk, and phenylalanine transporter (pheP).

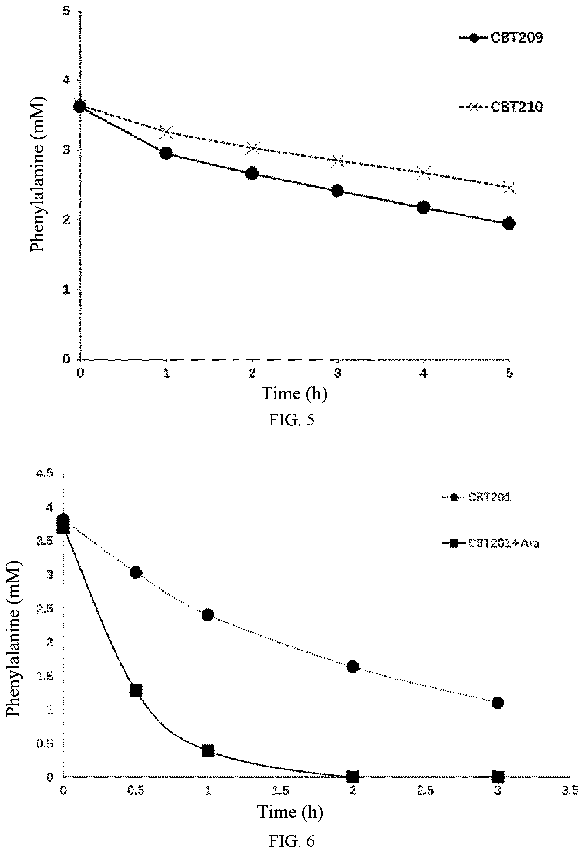

For comparison, this example also constructed the engineered strain CBT-210, which contains single copies of aromatic amino acid transaminase (TyrB), phenylpyruvate decarboxylase (Aro10), glutamate dehydrogenase (Gdh2), aldehyde reduction enzyme yahk and phenylalanine transporter (pheP), and phenylalanine aminotransferase (TyrB) was the only enzyme that directly converted phenylalanine. By comparing their in vitro metabolizing capabilities using the method in Example 5, the results are shown in Fig. 5 (comparing CBT-209 to CBT-210). The applicant unexpectedly found that the CBT-209 strain not only exhibited the capability of effective phenylalanine degradation, but its efficiency was even better than that of the CBT-210 strain, which degraded phenylalanine through aromatic amino acid transaminase. This finding further enriches the selection of enzymes for the first step of phenylalanine metabolism in engineered bacteria. Subsequently, considering the complexity of the environment and conditions faced by the engineered bacteria when used for in vivo therapy, the inventors constructed engineered bacteria containing both phenylalanine aminotransferase (TyrB) and phenylalanine dehydrogenase (pheDH) (e.g., the plurality of engineered bacteria constructed as described in 4.4 to 4.8 of Example 4), so as to fully utilize the advantages of different metabolizing enzymes in the phenylalanine metabolism pathway and improve the phenylalanine degradation effect of the engineered strains.

### 7.2 The addition of L-amino acid deaminase (LAAD) further improves the metabolism of phenylalanine

In addition to the engineered bacteria for phenylalanine aminotransferase (TyrB) and phenylalanine dehydrogenase (pheDH), in order to further improve the phenylalanine metabolism effect, the inventors further integrated single copy of L-amino acid deaminase (LAAD) into the genome of the engineered bacteria and carried out detection using the method in Example 5. The results are shown in Figs. 6 to 9. CBT-201, CBT-202, CBT-205, CBT-206, CBT-207 and CBT-208 strains were already able to degrade phenylalanine at a high efficiency without inducing LAAD expression by arabinose (Ara), which suggested that the use of phenylalanine dehydrogenase and/or phenylalanine aminotransferase may achieve good metabolic effects. However, the strain showed a stronger ability to metabolize phenylalanine after expressing the phenylalanine deaminase LAAD through the induction of arabinose.

### 7.3 Knocking out flagellum-related genes would not affect the ability of engineered bacteria to metabolize phenylalanine

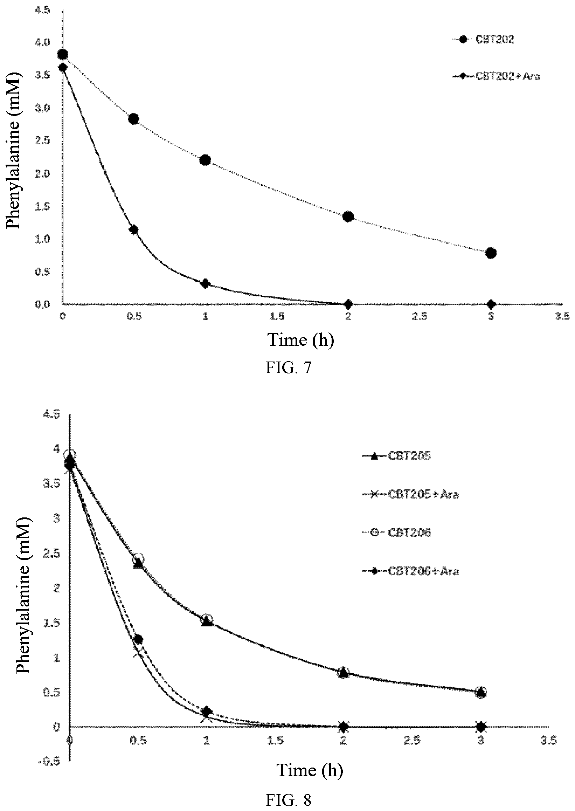

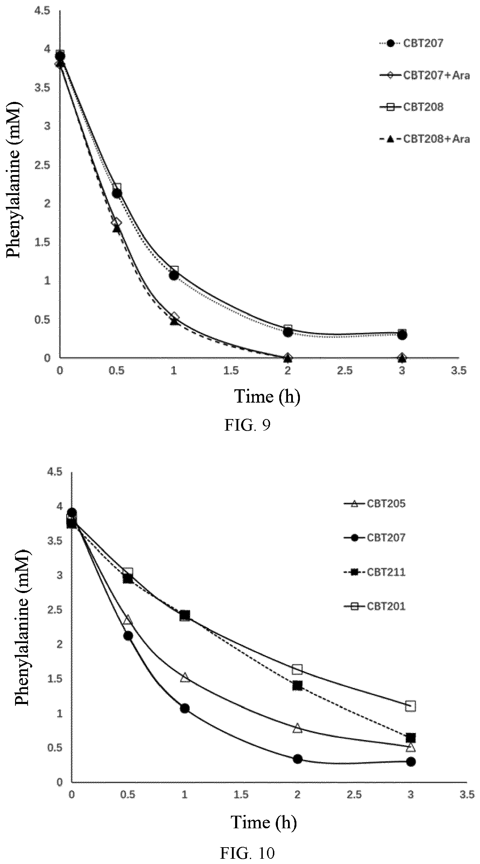

The flagellum is associated with the motility of E. coli. It has been shown that knocking out the flagellar synthesis/structure-related genes of E. coli will cause defects in the flagellar structure of E. coli, thereby affecting its motility and increasing the residence time of E. coli in the body (Zachary J. S. Mays, Todd C. Chappell, and Nikhil U. Nair ACS Synthetic Biology 2020 9 (2), 356-367. DOI: 10.1021/acssynbio.9b00356). Therefore, knocking out flagellum-related genes in the genome of engineered bacteria is expected to be an effective means to prolong the residence time of phenylalanine-metabolizing engineered bacteria in the intestinal tracts of patients, thereby enhancing the persistence of phenylalanine degradation. Therefore, this example used the method in Example 5 to test whether the knockout of flagellum-related genes in an in vitro environment has an adverse effect on the ability of engineered bacteria to degrade phenylalanine. The results are shown in Figs. 8 and 9. Both CBT-205 strain and CBT-207 strain were engineered bacteria with normal flagellar function, while CBT-206 strain and CBT-208 strain both had the flagellum-related genes bscA and fliC knocked out. As can be seen from the results (comparing CBT-205 to CBT-206 in Fig. 8, and CBT-207 to CBT-208 in Fig. 9, respectively), the knockout of flagellum-related genes did not affect the function and activity of the strains.

### 7.4 The Pvan promoter could confer stronger metabolic activity to the engineered bacteria than the Pfnrs promoter

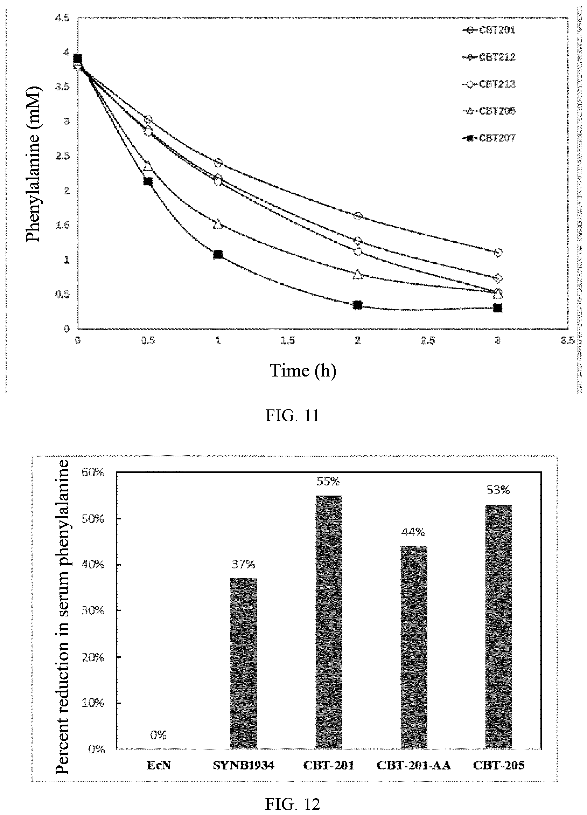

This example used the method in Example 5 to test the effect of the Pvan promoter on the metabolic ability of the engineered bacteria compared to the Pfnrs promoter. CBT-201 and CBT-211 were transformed with the same enzyme and number of copies, and the difference was only that all expression cassettes/copies in CBT-201 used the PfnrS promoter, while all expression cassettes/copies in CBT-211 used the Pvan Promoter, and the results are shown in Fig. 10 (i.e., the two curves at the top), with the activity of CBT-211 being higher than that of CBT-201. In addition, it can also be seen from Fig. 11 that CBT-205 and CBT-212 were also transformed with the same enzyme and expression cassette copy number. CBT-205 used 6 Pvan promoters and CBT-212 used 2 Pvan promoters and 4 PfnrS promoters. As a result, the activity of CBT-205, which used Pvan promoters exclusively, was higher than that of CBT-212, which used both Pvan and PfnrS promoters (comparing CBT-205 to CBT-212); The same phenomenon occurred in the strains CBT-207 and CBT-213 (see also Fig. 11, comparing CBT-207 to CBT-213). The above results show that the Pfnrs promoter has shown satisfactory activity, and the Pvan promoter can confer stronger in vitro metabolic activity to the engineered bacteria than the Pfnrs promoter.

### 7.5 Increasing the copy number of a specific expression cassette could improve the metabolic activity of the strains

This example used the method in Example 5 to test the effect of the copy number of different expression cassettes on the metabolic ability of the engineered bacteria. Both CBT-205 and CBT-207 used a better Pvan promoter. CBT-207 had one copy of pheDH expression cassette added therein on the basis of CBT-205. As can be seen from Fig. 10 (compare CBT-207 to CBT-205), the metabolic efficiency of CBT-207 is significantly improved compared to CBT-205.

### Comparative example 1: Construction of engineered bacterium SYNB1934 for phenylalanine metabolism

SYNB1934, a phenylalanine metabolizing engineered strain under development by Synlogic, was also constructed with EcN as the chassis bacterium, and is currently in Phase III clinical studies. According to previously reported data, SYBN1934 has demonstrated good metabolic activity in both animals and humans. In this comparative example, SYNB1934 was constructed according to the method published by Synlogic (for example, Isabella, V. M., B. N. Ha, M. J. Castillo, D. J. Lubkowicz, S. E. Rowe, Y. A. Millet, C. L. Anderson, N. Li, A. B. Fisher, K. A. West, P. J. Reeder, M. M. Momin, C. G. Bergeron, S. E. Guilmain, P. F. Miller, C. B. Kurtz and D. Falb (2018). "Development of a synthetic live bacterial therapeutic for the human metabolic disease phenylketonuria." Nat Biotechnol 36(9): 857-864. and Adolfsen, K. J., I. Callihan, C. E. Monahan, P. J. Greisen, J. Spoonamore, M. Momin, L. E. Fitch, M. J. Castillo, L. Weng, L. Renaud, C. J. Weile, J. H. Konieczka, T. Mirabella, A. Abin-Fuentes, A. G. Lawrence and V. M. Isabella (2021). "Improvement of a synthetic live bacterial therapeutic for phenylketonuria with biosensor-enabled enzyme engineering." Nat Commun 12(1): 6215.), and used as the activity reference of the present disclosure to compare the metabolic activity of the engineered bacteria to that of SYNB1934. The genotype information of SYNB1934 is as follows:
EcNΔrhtBC: :Ptac-phePΔaraDC: :Para-LAADΔ05580: :Ptac-stlA1934ΔmalEK: :Ptac-stlA1 934ΔagaI::Ptac-stlA1934ΔyicS/nepI::Ptac-stlAΔdapA

### Example 8: In vivo activity experiment of EcN and engineered strains

Phenylketonuria mice Pah KO (referred to as PKU mice) were provided by Jiangsu Gempharmatech Co., Ltd.. Newborn phenylketonuria mice were fed with phenylalanine-free (Phe-free) feed, and drinking water containing phenylalanine (0.2 g/L) was used as phenylalanine supplement. Mice older than 8 weeks could be used to test the ability of engineered bacteria to degrade phenylalanine. 2 h before the start of the animal experiment (T = -2 h), the supply of phenylalanine-containing drinking water was stopped and replaced with ordinary drinking water, and serum was collected to detect the phenylalanine concentration in the blood. At the beginning of the experiment (T = 0), mice were subcutaneously injected with 0.05 mg/g (BW) phenylalanine solution (5 mg/ml), and 200 µL (5 × 10¹⁰ cfu/mouse) of probiotic EcN or engineered bacteria (the engineered bacteria included engineered bacteria CBT-201, CBT-201-AA, and CBT-205 of the present disclosure and SYNB1934 constructed in Comparative Example 1 as an active reference) were used for gavage 1, 2, and 3 h after the subcutaneous injection of phenylalanine, with 5 mice per group. 4 h after the injection of phenylalanine, serum was collected to detect the concentration of phenylalanine in the blood.

The changes in the concentration of phenylalanine in the blood of mice are shown in Fig. 12. The values of increase in the concentration of phenylalanine in the blood of mice gavaged with all the selected engineered bacteria were significantly lower than those of mice gavaged with EcN, indicating that the engineered bacteria were able to effectively control the concentration of serum phenylalanine. Compared to mice gavaged with EcN, mice gavaged with CBT-201 engineered bacteria had a 55% reduction of phenylalanine in the blood, and their phenylalanine degradation capability was approximately 50% higher than that of the SYNB1934 engineered bacteria, which served as an active reference (SYNB1934 engineered bacteria were able to achieve a 37% reduction in phenylalanine concentration). In addition, the phenylalanine concentrations of CBT-201-AA and CBT-205 were reduced by 44% and 53.1% respectively, both of which were significantly higher than that of the SYNB1934 engineered bacteria. It can be seen that the engineered bacteria constructed according to the present disclosure exhibited an extremely strong phenylalanine metabolizing activity in vivo. In addition, compared to CBT-201, CBT-201-AA added two copies of the Pfnrs-ARO10-yahk expression cassette, but the reduction in phenylalanine concentration was slightly decreased, which also indicated that the copy number/expression of the relevant enzymes in the phenylalanine metabolic pathway in the transformed engineered bacteria was not the more the better.

In summary, the engineered bacteria constructed in the present disclosure effectively degrade phenylalanine both in vitro and in vivo, and can be used to treat phenylketonuria. The engineered bacterium constructed in the present disclosure can effectively degrade phenylalanine and/or phenylpyruvate. Experiments have proven that the engineered bacterium effectively degraded phenylalanine and phenylalanine metabolites in the body of PKU mice, showing good application prospects.

The above descriptions are only examples of the present disclosure, and do not limit the scope of the present disclosure; Any transformation to equivalent structures or equivalent procedures made using the contents of the description and the accompanying drawings of the present disclosure or direct or indirect application of the contents of the description and the accompanying drawings of the present disclosure in other relevant technical fields should likewise be similarly encompassed in the scope of the protection of the present disclosure.

## Claims

1. An engineered microorganism, including the following exogenous genes:
one or more genes encoding an enzyme capable of converting phenylalanine to phenylpyruvate;
one or more genes encoding an enzyme capable of converting phenylpyruvate to phenylacetaldehyde;
one or more genes encoding an enzyme capable of converting phenylacetaldehyde to phenylethanol; and
one or more genes encoding a protein capable of transporting phenylalanine into the engineered microorganism.

2. The engineered microorganism of claim 1, wherein the engineered microorganism is capable of metabolizing phenylalanine in an intestine in human and/or mammal.

3. The engineered microorganism of claim 1 or 2, wherein the exogenous gene is integrated into the genome or located on an expression plasmid.

4. The engineered microorganism of any one of claims 1 to 3, wherein the gene encoding the enzyme capable of converting phenylalanine to phenylpyruvate is selected from one or more of a gene encoding transaminase, a gene encoding dehydrogenase, a gene encoding deaminase, and functional equivalents thereof, and the functional equivalents retain at least part of the activities of the related enzymes.

5. The engineered microorganism of claim 4, wherein the gene encoding dehydrogenase is a gene encoding phenylalanine dehydrogenase.

6. The engineered microorganism of claim 5, wherein the gene encoding phenylalanine dehydrogenase is derived from a virus, a fungus, and/or a bacterium.

7. The engineered microorganism of claim 6, wherein the gene encoding phenylalanine dehydrogenase is derived from *Sporosarcina ureae* and/or *Bacillus,* such as *Sporosarcina ureae* SCRC-R04, *Lysinibacillus sphaericus* SCRC-R79a, *Bacillus badius,* and/or *Bacillus sp.* SLBN-3.

8. The engineered microorganism of claim 7, wherein the gene encoding phenylalanine dehydrogenase is selected from PheDH (Phenylalanine Dehydrogenase) of *Bacillus sp.* SLBN-3.

9. The engineered microorganism of claim 8, wherein the phenylalanine dehydrogenase has an amino acid sequence as shown in SEQ ID NO: 6; or the gene encoding the phenylalanine dehydrogenase has a nucleotide sequence as shown in SEQ ID NO: 17.

10. The engineered microorganism of any one of claims 4-9, wherein the gene encoding transaminase is a gene encoding aromatic amino acid transaminase.

11. The engineered microorganism of claim 10, wherein the gene encoding aromatic amino acid transaminase is derived from a virus, a fungus, and/or a bacterium.

12. The engineered microorganism of claim 11, wherein the gene encoding aromatic amino acid transaminase is derived from *Escherichia coli* and/or a yeast, such as *Escherichia coli* BL21 (DE3) and/or *Saccharomyces cerevisiae* S288C.

13. The engineered microorganism of claim 12, wherein the gene encoding aromatic amino acid transaminase is selected from TyrB (Tyrosine aminotransferase) of *Escherichia coli* BL21 (DE3) or ARO8 (Bifunctional 2-aminoadipate transaminase/aromatic-amino-acid: 2-oxoglutarate transaminase) of *Saccharomyces cerevisiae* S288C.

14. The engineered microorganism of claim 13, wherein the aromatic amino acid transaminase has an amino acid sequence as shown in SEQ ID NO: 1 or SEQ ID NO: 2; or the gene encoding the aromatic amino acid transaminase has a nucleotide sequence as shown in SEQ ID NO: 15 or SEQ ID NO: 90.

15. The engineered microorganism of any one of claims 10-14, wherein the engineered microorganism further includes one or more of a gene encoding glutamate dehydrogenase and functional equivalents thereof, and the functional equivalents retain at least part of the activities of the related enzymes.

16. The engineered microorganism of claim 15, wherein the gene encoding glutamate dehydrogenase is derived from a virus, a fungus, and/or a bacterium.

17. The engineered microorganism of claim 16, wherein the gene encoding glutamate dehydrogenase is derived from a yeast and/or *Clostridioides difficile,* such as *Saccharomyces cerevisiae* S288C and/or *Clostridioides difficile.*

18. The engineered microorganism of claim 17, wherein the gene encoding glutamate dehydrogenase is selected from GDH2 (Glutamate dehydrogenase (NAD+)) of *Saccharomyces cerevisiae* S288C or *Clostridioides difficile.*

19. The engineered microorganism of claim 18, wherein the glutamate dehydrogenase has an amino acid sequence as shown in SEQ ID NO: 11 or SEQ ID NO: 12; or the gene encoding the glutamate dehydrogenase has a nucleotide sequence as shown in SEQ ID NO: 20 or SEQ ID NO: 97.

20. The engineered microorganism of any one of claims 4 to 19, wherein the gene encoding deaminase is a gene encoding L-amino acid deaminase.

21. The engineered microorganism of claim 20, wherein the gene encoding L-amino acid deaminase is derived from a virus, a fungus, and/or a bacterium.

22. The engineered microorganism of claim 21, wherein the gene encoding L-amino acid deaminase is derived from *Escherichia coli,* a yeast, and/or *Proteus mirabilis,* such as *Escherichia coli* BL21 (DE3), *Saccharomyces cerevisiae* S288C, and/or *Proteus mirabilis* HI4320.

23. The engineered microorganism of claim 22, wherein the gene encoding L-amino acid deaminase is selected from LAAD (L-amino acid deaminase) of *Escherichia coli* BL21 (DE3), *Saccharomyces cerevisiae* S288C, or *Proteus mirabilis* HI4320.

24. The engineered microorganism of claim 23, wherein the L-amino acid deaminase has an amino acid sequence as shown in SEQ ID NO: 89, or the gene encoding the L-amino acid deaminase has a nucleotide sequence as shown in SEQ ID NO: 91.

25. The engineered microorganism of any one of claims 4-24, wherein the gene encoding the enzyme capable of converting phenylalanine to phenylpyruvate is selected from the following group:
(1) a gene encoding phenylalanine dehydrogenase;
(2) a gene encoding aromatic amino acid transaminase;
(3) a gene encoding aromatic amino acid transaminase and a gene encoding phenylalanine dehydrogenase;
(4) a gene encoding phenylalanine dehydrogenase and a gene encoding L-amino acid deaminase;
(5) a gene encoding aromatic amino acid transaminase and a gene encoding L-amino acid deaminase; or
(6) a gene encoding aromatic amino acid transaminase, a gene encoding phenylalanine dehydrogenase, and a gene encoding L-amino acid deaminase.

26. The engineered microorganism of any one of claims 1-25, wherein the gene encoding the enzyme capable of converting phenylpyruvate to phenylacetaldehyde is selected from one or more of a gene encoding phenylpyruvate decarboxylase, a gene encoding α-keto acid decarboxylase, and functional equivalents thereof, and the functional equivalents retain at least part of the activities of the related enzymes.

27. The engineered microorganism of claim 26, wherein the gene encoding phenylpyruvate decarboxylase is derived from a virus, a fungus, and/or a bacterium.

28. The engineered microorganism of claim 27, wherein the gene encoding phenylpyruvate decarboxylase is derived from a yeast, such as *Saccharomyces cerevisiae* S288C.

29. The engineered microorganism of claim 28, wherein the gene encoding phenylpyruvate decarboxylase is ARO10 (Phenylpyruvate decarboxylase) of *Saccharomyces cerevisiae* S288C.

30. The engineered microorganism of claim 29, wherein the phenylpyruvate decarboxylase has an amino acid sequence as shown in SEQ ID NO: 7; or the gene encoding the phenylpyruvate decarboxylase has a nucleotide sequence as shown in SEQ ID NO: 18.

31. The engineered microorganism of claim 26, wherein the gene encoding α-keto acid decarboxylase is derived from a virus, a fungus, and/or a bacterium.

32. The engineered microorganism of claim 31, wherein the gene encoding α-keto acid decarboxylase is derived from a yeast and/or *Proteus mirabilis,* such as *Saccharomyces cerevisiae* S288C and/or *Proteus mirabilis* JN458.

33. The engineered microorganism of claim 32, wherein the gene encoding α-keto acid decarboxylase is selected from at least one of KDC (α-keto-acid decarboxylase) of *Saccharomyces cerevisiae* S288C or *Proteus mirabilis* JN458.

34. The engineered microorganism of claim 33, wherein the α-keto acid decarboxylase has a sequence as shown in SEQ ID NO:8; or the gene encoding the α-keto acid decarboxylase has a nucleotide sequence as shown in SEQ ID NO: 95.

35. The engineered microorganism of any one of claims 1 to 34, wherein the gene encoding the enzyme capable of converting phenylacetaldehyde to phenylethanol is selected from one or more of a gene encoding aldehyde reductase and functional equivalents thereof, and the functional equivalents retain at least part of the activities of the related enzymes.

36. The engineered microorganism of claim 35, wherein the gene encoding aldehyde reductase is derived from a virus, a fungus, and/or a bacterium.

37. The engineered microorganism of claim 36, wherein the gene encoding aldehyde reductase is derived from *Escherichia coli* and/or *Lactobacillus brevis,* such as *Escherichia coli* str. K-12 substr. MG1655 and/or *Lactobacillus brevis.*

38. The engineered microorganism of claim 37, wherein the gene encoding aldehyde reductase is selected from YahK (NADPH-dependent aldehyde reductase) of *Escherichia coli* str. K-12 substr. MG1655 or ADH (Alcohol dehydrogenase) of *Lactobacillus brevis.*

39. The engineered microorganism of claim 38, wherein the aldehyde reductase has an amino acid sequence as shown in SEQ ID NO: 9 or SEQ ID NO: 10; or the gene encoding the aldehyde reductase has a nucleotide sequence as shown in SEQ ID NO: 19 or SEQ ID NO: 96.

40. The engineered microorganism of any one of claims 1 to 39, wherein the gene encoding the protein capable of transporting phenylalanine into the engineered microorganism is selected from one or more of a gene encoding phenylalanine transporter and functional equivalents thereof, and the functional equivalents retain at least part of the activities of the related enzymes.

41. The engineered microorganism of claim 40, wherein the gene encoding phenylalanine transporter is derived from a virus, a fungus, and/or a bacterium.

42. The engineered microorganism of claim 41, wherein the gene encoding phenylalanine transporter is derived from *E. coli.*

43. The engineered microorganism of claim 42, wherein the gene encoding phenylalanine transporter is PheP (Phenylalanine: H(+) symporter) of *E. coli.*

44. The engineered microorganism of claim 43, wherein the phenylalanine transporter has an amino acid sequence as shown in SEQ ID NO: 13; or the gene encoding the phenylalanine transporter has a nucleotide sequence as shown in SEQ ID NO: 21.

45. The engineered microorganism of any one of claims 1-44, wherein any one, two, three, four, five, six, a plurality of or all of the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, the gene encoding glutamate dehydrogenase, and/or the gene encoding phenylalanine transporter are optionally operably linked to one or more of identical or different promoters.

46. The engineered microorganism of claim 45, wherein the gene encoding aromatic amino acid transaminase and the gene encoding glutamate dehydrogenase share the same promoter.

47. The engineered microorganism of claim 45, wherein the gene encoding phenylpyruvate decarboxylase and the gene encoding aldehyde reductase share the same promoter.

48. The engineered microorganism of claim 45, wherein the gene encoding phenylalanine dehydrogenase and the gene encoding phenylalanine transporter share the same promoter.

49. The engineered microorganism of claim 45, wherein the gene encoding L-amino acid deaminase alone is operably linked to a promoter.

50. The engineered microorganism of claim 45, wherein the gene encoding phenylalanine transporter alone is operably linked to a promoter.

51. The engineered microorganism of any one of claims 45-50, wherein the promoter is an endogenous promoter or an exogenous promoter.

52. The engineered microorganism of any one of claims 45-50, wherein the promoter is an inducible promoter or a constitutive promoter.

53. The engineered microorganism of claim 52, wherein the promoter is a directly or indirectly inducible promoter.

54. The engineered microorganism of claim 53, wherein the promoter is induced directly or indirectly by an exogenous environmental condition.

55. The engineered microorganism of claim 54, wherein the promoter is induced directly or indirectly by an exogenous environmental condition in the intestine in mammal.

56. The engineered microorganism of any one of claims 51-55, wherein the promoter is selected from at least one of PfnrS, FDHF, Ptet, Pbba, Ptrc, Pvan, or PBAD.

57. The engineered microorganism of claim 56, wherein the sequence of the PfnrS promoter is as shown in SEQ ID NO. 22.

58. The engineered microorganism of claim 56, wherein the sequence of the FDHF promoter is as shown in SEQ ID NO. 24.

59. The engineered microorganism of claim 56, wherein the sequence of the Ptet promoter is as shown in SEQ ID NO. 100.

60. The engineered microorganism of claim 56, wherein the sequence of the Pbba promoter is as shown in SEQ ID NO. 98.

61. The engineered microorganism of claim 56, wherein the sequence of the Ptrc promoter is as shown in SEQ ID NO. 99.

62. The engineered microorganism of claim 56, wherein the sequence of the Pvan promoter is as shown in SEQ ID NO: 101.

63. The engineered microorganism of claim 56, wherein the sequence of the PBAD promoter is as shown in SEQ ID NO: 102.

64. The engineered microorganism of any one of claims 1-63, wherein the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, the gene encoding glutamate dehydrogenase, and/or the gene encoding phenylalanine transporter are/is located within one or more expression cassettes.

65. The engineered microorganism of claim 64, wherein the engineered microorganism includes a first expression cassette and a second expression cassette, wherein the first expression cassette includes a first promoter, and the gene encoding aromatic amino acid transaminase and the gene encoding glutamate dehydrogenase operably linked to the first promoter; the second expression cassette includes a second promoter, and the gene encoding phenylpyruvate decarboxylase and the gene encoding aldehyde reductase operably linked to the second promoter; the first promoter or the second promoter may be identical or different.

66. The engineered microorganism of claim 65, wherein the engineered microorganism further includes a third expression cassette, wherein the third expression cassette includes the gene encoding phenylalanine transporter operably linked to a third promoter, and the third promoter may be identical to the first promoter and/or the second promoter, or may be different from both the first promoter and the second promoter.

67. The engineered microorganism of claim 64, wherein the engineered microorganism includes a fourth expression cassette and a second expression cassette, wherein the fourth expression cassette includes a fourth promoter, and the gene encoding phenylalanine dehydrogenase and the gene encoding phenylalanine transporter operably linked to the fourth promoter; the second expression cassette includes a second promoter, and the gene encoding phenylpyruvate decarboxylase and the gene encoding aldehyde reductase operably linked to the second promoter; the fourth promoter or the second promoter may be identical or different.

68. The engineered microorganism of claim 67, wherein the engineered microorganism further includes a first expression cassette, wherein the first expression cassette includes a first promoter, and the gene encoding aromatic amino acid transaminase and the gene encoding glutamate dehydrogenase operably linked to the first promoter; the first promoter may be the identical to the fourth promoter and/or the second promoter, or may be different from both the fourth promoter and the second promoter.

69. The engineered microorganism of claim 67 or 68, wherein the engineered microorganism further includes a fifth expression cassette, wherein the fifth expression cassette includes a fifth promoter, and the gene encoding L-amino acid deaminase operably linked to the fifth promoter; the fifth promoter is identical to or different from the fourth promoter, the second promoter and/or the first promoter.

70. The engineered microorganism of any one of claims 64-69, wherein all or part of the first promoter, the second promoter, the third promoter, and the fourth promoter are selected from at least one of PfnrS and/or Pvan, and the fifth promoter is PBAD.

71. The engineered microorganism of any one of claims 64-69, wherein the expression cassette is present in the form of single copy, two copies, three copies, four copies or more copies.

72. The engineered microorganism of claim 71, wherein the first expression cassette is present in the form of single copy or two copies.

73. The engineered microorganism of claim 71, wherein the second expression cassette is present in the form of single copy, two copies, three copies, or four copies.

74. The engineered microorganism of claim 71, wherein the third expression cassette is present in the form of single copy.

75. The engineered microorganism of claim 71, wherein the fourth expression cassette is present in the form of single copy or two copies.

76. The engineered microorganism of claim 71, wherein the fifth expression cassette is present in the form of single copy.

77. The engineered microorganism of any one of claims 64-76, wherein the expression cassette is located on a plasmid.

78. The engineered microorganism of any one of claims 64-76, wherein the expression cassette is stably integrated in one, two, or more identical or different genomic sites of the engineered microorganism.

79. The engineered microorganism of claim 78, wherein the genomic site is selected from at least one of yicS site, malPT site, malE site, exo site, rhtB/C site, agaI/rsml site, araBD site, yghx site, ldhA site, araAB site, lacZ site, kefB site, maeB site, nth/tppB site, and/or tkrA site.

80. The engineered microorganism of claim 79, wherein the first expression cassette is integrated at the yghx site and/or the araAB site.

81. The engineered microorganism of claim 79, wherein the second expression cassette is integrated at the ldhA site, the lacZ site, the yghx site, the yjcS site and/or the agaI/rsml site.

82. The engineered microorganism of claim 79, wherein the third expression cassette is integrated at the kefB site.

83. The engineered microorganism of claim 79, wherein the fourth expression cassette is integrated at the kefB site, the ldhA site, the nth/tppB site, the maeB site and/or the tkrA site.

84. The engineered microorganism of claim 79, wherein the fifth expression cassette is integrated at the rhtB/C site.

85. The engineered microorganism of any one of the preceding claims, including:
1) single copy of the fourth expression cassette, wherein the fourth expression cassette is integrated at the kefB site; two copies of the second expression cassette, wherein the second expression cassette is integrated at the ldhA site and the lacZ site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the PfnrS promoter, and the promoter used in the fifth expression cassette is the PBAD promoter;
2) single copy of the fourth expression cassette, wherein the fourth expression cassette is integrated at the nth/tppB site; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the Pvan promoter, and the promoter used in the fifth expression cassette is the PBAD promoter;
3) two copies of the fourth expression cassette, wherein the fourth expression cassette is integrated at the nth/tppB site and the tkrA site respectively; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the Pvan promoter, and the promoter used in the fifth expression cassette is the PBAD promoter;
4) single copy of the fourth expression cassette, wherein the fourth expression cassette is integrated at the maeB site; three copies of the second expression cassette, wherein the second expression cassette is integrated at the yjcS site, the lacZ site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the single copy of the fourth expression cassette uses the PfnrS promoter, the three copies of the second expression cassette use the PfnrS promoter, the PfnrS promoter and the Pvan promoter respectively, the two copies of the first expression cassette use the PfnrS promoter and the Pvan promoter respectively, and the promoter used in the fifth expression cassette is the PBAD promoter;
5) two copies of the fourth expression cassette, wherein the fourth expression cassette is integrated at the maeB site and the kefB site; three copies of the second expression cassette, wherein the second expression cassette is integrated at the yjcS site, the ldhA site and the agaI/rsml site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the two copies of the fourth expression cassette use the PfnrS promoter, the three copies of the second expression cassette use the Pvan promoter, the Pvan promoter and the PfnrS promoter respectively, the two copies of the first expression cassette use the Pvan promoter, and the promoter used in the fifth expression cassette is the PBAD promoter;
6) single copy of the fourth expression cassette, wherein the fourth expression cassette is integrated at the kefB site; four copies of the second expression cassette, wherein the second expression cassette is integrated at the ldhA site, the lacZ site, the agaI/rsml site and the yjcS site respectively; two copies of the first expression cassette, wherein the first expression cassette is integrated at the yghX site and the araAB site respectively; and single copy of the fifth expression cassette, wherein the fifth expression cassette is integrated at the rhtB/C site; among them, the promoter used in the fourth, second and first expression cassettes is the PfnrS promoter, and the promoter used in the fifth expression cassette is the PBAD promoter;
7) two copies of the fourth expression cassette, wherein the fourth expression cassette is integrated at the ldhA site and the maeB site; two copies of the second expression cassette, wherein the second expression cassette is integrated at the yghX site and the lacZ site respectively; and single copy of the fifth expression cassette, wherein the fifth expression cassette is integrated at the rhtB/C site; among them, the promoter used in the fourth and second expression cassettes is the PfnrS promoter, and the promoter used in the fifth expression cassette is the PBAD promoter;
8) single copy of the fourth expression cassette, wherein the fourth expression cassette is integrated at the ldhA site; and single copy of the second expression cassette, wherein the second expression cassette is integrated at the yghX site; among them, the promoter used in the fourth and second expression cassettes is the PfnrS promoter;
9) single copy of the first expression cassette, wherein the first expression cassette is integrated at the yghX site; single copy of the second expression cassette, wherein the second expression cassette is integrated at the ldhA site; and single copy of the third expression cassette, wherein the third expression cassette is integrated at the kefB site; among them, the promoter used in the first, second and third expression cassettes is the PfnrS promoter;
10) single copy of the first expression cassette, wherein the first expression cassette is integrated at the yghX site; and single copy of the second expression cassette, wherein the second expression cassette is integrated at the ldhA site; among them, the promoter used in the first and second expression cassettes is the PfnrS promoter;
11) two copies of the first expression cassette, wherein the first expression cassette is integrated at the yghX site and the araAB site respectively; and two copies of the second expression cassette, wherein the second expression cassette is integrated at the ldhA site and the lacZ site; among them, the promoter used in the first and second expression cassettes is the PfnrS promoter; or
12) two copies of the first expression cassette, wherein the first expression cassette is integrated at the yghX site and the araAB site respectively; two copies of the second expression cassette, wherein the second expression cassette is integrated at the ldhA site and the lacZ site respectively; and single copy of the third expression cassette, wherein the third expression cassette is integrated at the kefB site; among them, the promoter used in the first, second and third expression cassettes is the PfnrS promoter.

86. The engineered microorganism of any one of the preceding claims, wherein the engineered microorganism is a bacterium or a yeast.

87. The engineered microorganism of claim 86, wherein the engineered microorganism is a non-pathogenic bacterium.

88. The engineered microorganism of claim 87, wherein the engineered microorganism is a probiotic.

89. The engineered microorganism of claim 88, wherein the engineered microorganism is selected from at least one of *Bacteroides, Bifidobacterium, Clostridium, Escherichia, Lactobacillus* and *Lactococcus.*

90. The engineered microorganism of claim 89, wherein the engineered microorganism is *Escherichia coli.*

91. The engineered microorganism of claim 90, wherein the engineered microorganism is *Escherichia coli* strain Nissle1917.

92. The engineered microorganism of any one of claims 86-91, wherein the engineered microorganism exhibits auxotrophy.

93. The engineered microorganism of claim 92, wherein the auxotrophy is diaminoacrylic acid auxotrophy and/or thymine auxotrophy.

94. The engineered microorganism of claim 93, wherein the auxotrophy is compensated when the engineered microorganism is present in the intestine in mammal.

95. The engineered microorganism of claim 94, wherein the intestine in mammal is an intestine in human.

96. The engineered microorganism of any one of claims 86-95, wherein the flagellar function of the engineered microorganism is defective or lost.

97. The engineered microorganism of claim 96, wherein flagellar synthesis-related genes of the engineered microorganism have mutations or deletions.

98. The engineered microorganism of claim 97, wherein the flagellar synthesis-related genes are bscA and/or fliC genes.

99. An engineered microorganism composition, including more than one engineered microorganism, wherein each of the more than one engineered microorganism independently includes any one or more of the following genes:
a gene encoding an enzyme capable of converting phenylalanine to phenylpyruvate;
a gene encoding an enzyme capable of converting phenylpyruvate to phenylacetaldehyde;
a gene encoding an enzyme capable of converting phenylacetaldehyde to phenylethanol;
and a gene encoding a protein capable of transporting phenylalanine into the engineered microorganism;
wherein the engineered microorganism composition includes the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, and the gene encoding the protein capable of transporting phenylalanine into the engineered microorganism.

100. The engineered microorganism composition of claim 99, wherein each of the more than one engineered microorganism independently includes any one or more of the following genes:
a gene encoding an enzyme capable of converting phenylalanine to phenylpyruvate;
a gene encoding an enzyme capable of converting phenylpyruvate to phenylacetaldehyde;
a gene encoding an enzyme capable of converting phenylacetaldehyde to phenylethanol;
a gene encoding glutamate dehydrogenase;
and a gene encoding a protein capable of transporting phenylalanine into the engineered microorganism;
wherein the engineered microorganism composition includes the gene encoding the enzyme capable of converting phenylalanine to phenylpyruvate, the gene encoding the enzyme capable of converting phenylpyruvate to phenylacetaldehyde, the gene encoding the enzyme capable of converting phenylacetaldehyde to phenylethanol, the gene encoding glutamate dehydrogenase and the gene encoding the protein capable of transporting phenylalanine into the engineered microorganism.

101. A composition, including the engineered microorganism of any one of claims 1-98 or the engineered microorganism composition of claim 99 or 100 and a pharmaceutically, nutritionally or physiologically acceptable carrier.

102. The composition of claim 101, wherein the composition is a pharmaceutical composition.

103. The composition of claim 101, wherein the composition is an edible composition.

104. The composition of claim 101, wherein the composition is a probiotic composition.

105. The composition of claim 101, wherein the composition is a food supplement.

106. The composition of claim 101, wherein the composition is formulated for oral administration.

107. The composition of claim 101, wherein the composition is a liquid preparation, a solid preparation or a semi-solid preparation.

108. The composition of claim 107, wherein the liquid preparation is selected from at least one of a solution product or a suspension product.

109. The composition of claim 101, wherein the dosage form of the composition is selected the group of: powders, pulvises, tablets, dragees, capsules, granules, suspensions, solutions, syrups, drops, or sublingual tablets.

110. The composition of claim 101, wherein the composition is formulated for enteral administration, intrajejunal administration, intraduodenal administration, intraileal administration, gastric shunt administration or intracolonic administration via nanoparticles, nanocapsules, microcapsules or microtablets (which are enteric coated or uncoated).

111. A kit, including the engineered microorganism of any one of claims 1-98 or the engineered microorganism composition of claim 99 or 100, or the composition of any one of claims 101-110.

112. A method for alleviating and/or treating diseases and/or conditions associated with hyperphenylalaninemia, wherein the method includes administering the engineered microorganism of any one of claims 1-98, the engineered microorganism composition of claim 99 or 100, the composition of any one of claims 101-110, or the kit of claim 111 to a patient in need thereof.

113. The method of claim 112, wherein the diseases and/or conditions associated with hyperphenylalaninemia include: phenylketonuria (such as classical or typical phenylketonuria and atypical phenylketonuria), permanent mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.

114. Use of the engineered microorganism of any one of claims 1-98, the engineered microorganism composition of claim 99 or 100, or the composition of any one of claims 101-110 in the preparation of a drug or a health product for alleviating and/or treating diseases and/or conditions associated with hyperphenylalaninemia.

115. The use of claim 114, wherein the diseases and/or conditions associated with hyperphenylalaninemia include: phenylketonuria (such as classic or typical phenylketonuria and atypical phenylketonuria), permanent mild hyperphenylalaninemia, non-phenylketonuria types of hyperphenylalaninemia, phenylalanine hydroxylase deficiency, cofactor deficiency, dihydropterin reductase deficiency, tetrahydropterin synthase deficiency, Segawa's disease and liver diseases.
